# EUROPEAN PATENT APPLICATION

(11) **EP 3 090 754 A1**
(43) Date of publication of application: **09.11.2016**
(21) Application number: 16157727.5
(22) Date of filing: 30.12.2010
(51) Int. Cl.: A61K 38/07, A61P 9/10, A61P 13/12

(54) **METHODS FOR THE PREVENTION OR TREATMENT OF VESSEL OCCLUSION INJURY**

(30) Priority: 31.12.2009 US 291699 P; 09.07.2010 US 363138 P
(62) Divisional of application: 14197463.4
(71) Applicant: Stealth Peptides International, Inc., 98000 Monaco (MC)
(72) Inventor: BOROW, Kenneth, Bryn Mawr, PA Pennsylvania 19010 (US); WILSON, Travis D, Newton, MA Massachusetts 02461 (US)
(74) Representative: Atkinson, Jennifer

(57) **Abstract**

This invention provides methods of preventing or treating cardiac ischemiareperfusion injury in a mammalian subject. The methods comprise administering to the subject an effective amount of an aromatic-cationic peptide to a subject in need thereof, wherein the peptide is D-Arg-2 6-Dmt-Lys-Phe-NH2 (SS-31).

## Description

### CROSS-REFERENCE TO RELATED APPLICATIONS

This application claims priority to U.S. Provisional Patent Application No. 61/291,699, filed December 31, 2009, and U.S. Provisional Patent Application No. 61/363,138, filed July 9, 2010, the entire contents of which are hereby incorporated by reference in their entirety.

### TECHNICAL FIELD

The present technology relates generally to compositions and methods of preventing or treating vessel occlusion injury. In particular, embodiments of the present technology relate to administering aromatic-cationic peptides in effective amounts to prevent or treat acute myocardial infarction injury in mammalian subjects.

### BACKGROUND

The following description is provided to assist the understanding of the reader. None of the information provided or references cited is admitted to be prior art to the present invention.

Blood vessel occlusions are commonly treated by enhancing blood flow in the affected vessels. A variety of surgical and non-surgical interventional procedures have been developed over the years for opening stenosed or occluded blood vessels in a patient caused by the build up of plaque or other substances on the walls of the blood vessel, Such procedures usually involve the percutaneous introduction of the interventional device into the lumen of the artery, usually through a catheter. These revascularization procedures involve such devices as balloons, endovascular knives (atherectomy), and endovascular drills. The surgical approach is accompanied by significant morbidity and even mortality, while the angioplasty-type processes are complicated by recurrent stenoses in many cases.

Additional complications arise due to the restoration of blood flow to the ischemic tissues. This phenomenon is commonly referred to as reperfusion injury and may be more damaging to the tissue than ischemia. In particular, the absence of oxygen and nutrients typically delivered to the ischemic tissue region by the blood creates a condition in which the restoration of circulation results in inflammation and oxidative damage rather than restoration of normal function. This new supply of oxygen forms within cells which may damage cellular proteins, DNA and the plasma membrane. This may in turn cause the release of additional free radicals resulting in further cellular damage.

### SUMMARY

The present technology relates generally to the treatment or prevention of vessel occlusion injury in mammals through administration of therapeutically effective amounts of aromatic-cationic peptides to subjects in need thereof. The present technology also relates to the treatment or prevention of cardiac vessel occlusion injury in mammals through administration of therapeutically effective amounts of aromatic-cationic peptides to subjects in need thereof.

in one aspect, the disclosure provides (a) a method of treating or preventing a vessel occlusion injury, comprising administering to the subject a therapeutically effective amount of an aromatic-cationic peptide or a pharmaceutically acceptable salt thereof; and (b) performing a revascularization procedure on the subject. In some embodiments, the aromatic-cationic peptide is a peptide having:
at least one net positive charge;
a minimum of four amino acids;
a maximum of about twenty amino acids;
a relationship between the minimum number of net positive charges (pₘ) and the total number of amino acid residues (r) wherein 3pₘ is the largest number that is less than or equal to r + 1; and a relationship between the minimum number of aromatic groups (a) and the total number of net positive charges (pₜ) wherein 2a is the largest number that is less than or equal to pₜ + 1, except that when a is 1, pₜ may also be 1. In particular embodiments, the mammalian subject is a human.

In one embodiment, 2pₘ is the largest number that is less than or equal to r+1, and a may be equal to pₜ. The aromatic-cationic peptide may be a water-soluble peptide having a minimum of two or a minimum of three positive charges,

in one embodiment, the peptide comprises one or more non-naturally occurring amino acids, for example, one or more D-amino acids. In some embodiments, the C-terminal carboxyl group of the amino acid at the C-terminus is amidated. In certain embodiments, the peptide has a minimum of four amino acids. The peptide may have a maximum of about 6, a maximum of about 9, or a maximum of about 12 amino acids.

In one embodiment, the peptide comprises a tyrosine or a 2',6'-dimethyltyrosine (Dmt) residue at the N-terminus. For example, the peptide may have the formula Tyr-D-Arg-Phe-Lys-NH₂ or 2',6'-Dmt-D-Arg-Phe-Lys-NH₂. In another embodiment, the peptide comprises a phenylalanine or a 2',6'-dimethylphenylalanine residue at the N-terminus. For example, the peptide may have the formula Phe-D-Arg-Phe-Lys-NH₂ or 2',6'-Dmp-D-Arg-Phe-Lys-NH₂. In a particular embodiment, the aromatic-cationic peptide has the formula D-Arg-2',6'-Dmt-Lys-Phe-NH₂(also known as SS-31).

In one embodiment, the peptide is defined by formula I: 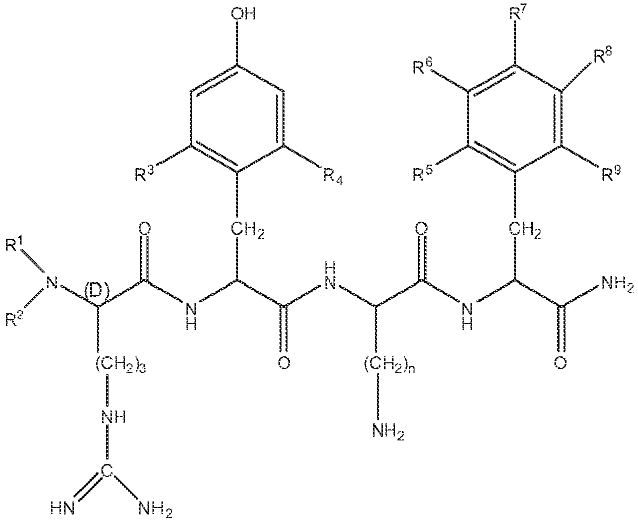
wherein R¹ and R² are each independently selected from
   (i) hydrogen;
   (ii) linear or branched C₁-G₆ alkyl;
   (iii) 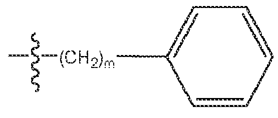 where m = 1-3;
   (iv) 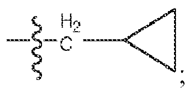
   (v) 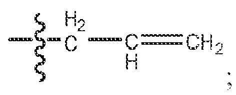
R³ and R⁴ are each independently selected from
   (i) hydrogen;
   (ii) linear or branched C₁-G₆ alkyl;
   (iii) C₁-G₆ alkoxy;
   (iv) amino;
   (v) C₁-C₄ alkylamino;
   (vi) C₁-C₄dialkylamino;
   (vii) nitro;
   (viii) hydroxyl;
   (ix) halogen, where "halogen" encompasses chloro, fluoro, bromo, and iodo;
R⁵, R⁶, R⁷, R⁸, and R⁹ are each independently selected from
   (i) hydrogen;
   (ii) linear or branched C₁-C₆ alkyl;
   (iii) C₁-G₆ alkoxy;
   (iv) amino;
   (v) C₁-C₄ alkylamino;
   (vi) C₁-C₄ dialkylamino;
   (vii) nitro;
   (viii) hydroxyl;
   (ix) halogen, where "halogen" encompasses chloro, fluoro, bromo, and iodo; and n is an integer from 1 to 5.

In a particular embodiment, R¹ and R² are hydrogen; R³ and R⁴ are methyl; R⁵, R⁶, R⁷, R⁸, and R⁹ are all hydrogen; and n is 4.

In one embodiment, the peptide is defined by formula II: 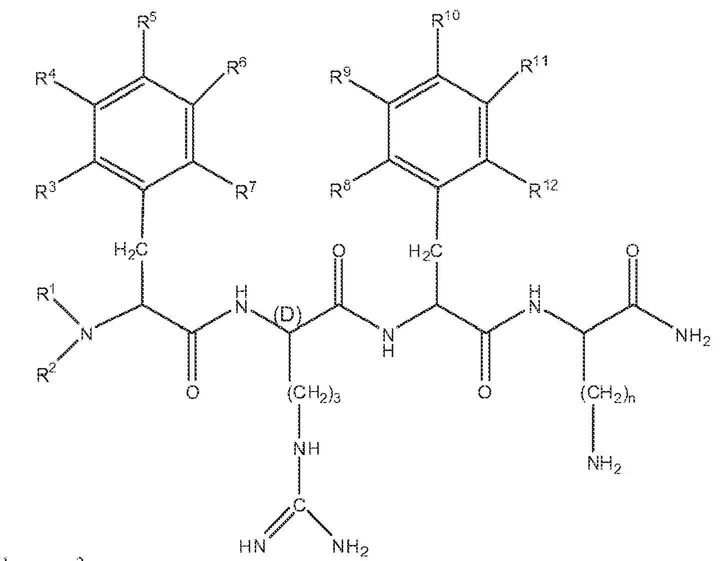
wherein R¹ and R² are each independently selected from
   (i) hydrogen;
   (ii) linear or branched C₁-G₆ alkyl;
   (iii) 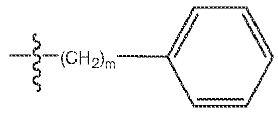 where m = 1-3;
   (iv) 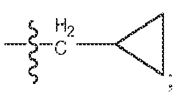
   (v) 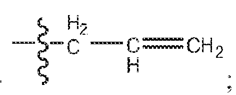
R³, R⁴, R⁵, R⁶, R⁷, R⁸, R⁹, R¹⁰, R¹¹ and R¹² are each independently selected from
   (i) hydrogen;
   (ii) linear or branched C₁-G₆ alkyl;
   (iii) C₁-C₆ alkoxy;
   (iv) amino;
   (v) C₁-C₄ alkylamino;
   (vi) C₁-C₄ dialkylamino;
   (vii) nitro;
   (viii) hydroxyl;
   (ix) halogen, where "halogen" encompasses chloro, fluoro, bromo, and iodo; and
   n is an integer from 1 to 5,

In a particular, embodiment, R¹, R², R³ R⁴, R⁵, R⁶, R⁷, R⁸, R⁹ R¹⁰, R¹¹ and R¹² are all hydrogen; and n is 4. In another embodiment, R¹, R², R³, R⁴, R⁵, R⁶, R⁷, R⁸, R⁹, and R¹¹ are all hydrogen; R⁸ and R¹² are methyl; R¹⁰ is hydroxyl; and n is 4.

The aromatic-cationic peptides may be administered in a variety of ways. In some embodiments, the peptides maybe administered orally, topically, intranasally, intraperitoneally, intravenously, subcutaneously, or transdermally (e*.g..* by iontophoresis).

In one embodiment, the subject is administered the peptide prior to ischemia. In one embodiment, the subject is administered the peptide prior to the reperfusion of ischemic tissue. In one embodiment, the subject is administered the peptide at about the time of reperfusion of ischemic tissue. In one embodiment, the subject is administered the peptide after reperfusion of ischemic tissue.

In one embodiment, the subject is administered the peptide prior to the revascularization procedure. In another embodiment, the subject is administered the peptide after the revascularization procedure. In another embodiment, the subject is administered the peptide during and after the revascularization procedure. In yet another embodiment, the subject is administered the peptide continuously before, during, and after the revascularization procedure.

In one embodiment, the subject is administered the peptide starting at least 5 minutes, at least 10 min, at least 30 min, at least 1 hour, at least 3 hours, at least 5 hours, at least 8 hours, at least 12 hours, or at least 24 hours prior to revascularization, *i*.*e*., reperfusion of ischemic tissue. In one embodiment, the subject is administered the peptide starting at about 5-30 min, from about 10-60 minutes, from about 10-90 min, or from about 10-120 min prior to the revascularization procedure. In one embodiment, the subject is administered the peptide until about 5-30 min, until about 10-60 min, until about 10-90 min, until about 10-120 min. or until about 10-180 min after the revascularization procedure.

In one embodiment, the subject is administered the peptide for at least 30 min, at least 1 hour, at least 3 hours, at least 5 hours, at least 8 hours, at least 12 hours, or at least 24 hours after the revascularization procedure, *i*.*e*., reperfusion of ischemic tissue. In one embodiment, the duration of administration of the peptide is about 30 min, about 1 hour, about 2 hours, about 3 hours, about 4 hours, about 5 hours, about 8 hours, about 12 hours, or about 24 hours after the revascularization procedure, *i*.*e*., reperfusion of ischemic tissue,

In one embodiment, the subject is administered the peptide as an IV infusion starting at about 1 min to 30 min prior to reperfusion (*i.e.* about 5 min, about 10 min, about 20 min, or about 30 min prior to reperfusion) and continuing for about 1 hour to 24 hours after reperfusion (*i.e*., about 1 hour, about 2 hours, about 3 hours, or about 4 hours after reperfusion). In one embodiment, the subject receives in IV bolus injection prior to reperfusion of the tissue. In one embodiment, the subject continues to receive the peptide chronically after the reperfusion period, *i.e.* for about 1-7 days, about 1-14 days, about 1-30 days after the reperfusion period. During this period, the peptide may be administered by any route, *e.g..* subcutaneously or intravenously.

In various embodiments, the subject is suffering from a myocardial infarction, a stroke, or is in need of angioplasty. In one embodiment, the revascularization procedure is selected from the group consisting of: balloon angioplasty; insertion of a stent; percutaneous coronary intervention (PCI), percutaneous transluminal coronary angioplasty; or directional coronary atherectomy. In one embodiment, the revascularization procedure is removal of the occlusion. In one embodiment, the revascularization procedure is administration of one or more thrombolytic agents. In one embodiment, the one or more thrombolytic agents are selected from the group consisting of: tissue plasminogen activator; urokinase; prourokinase; streptokinase; acylated form of plasminogen; acylated form of plasmin; and acylated streptokinase-plasminogen complex.

In one embodiment, the vessel occlusion is a cardiac vessel occlusion. In another embodiment, the vessel occlusion is an intracranial vessel occlusion. In yet other embodiments, the vessel occlusion is selected from the group consisting of: deep venous thrombosis; peripheral thrombosis; embolic thrombosis; hepatic vein thrombosis; sinus thrombosis; venous thrombosis; an occluded arterio-venal shunt; and an occluded catheter device,

Aspects of the invention may also be provided by the following numbered paragraphs
1. A method for treating a vessel occlusion injury in a mammalian subjects, the method comprising:
   (a) administering to the subject a therapeutically effective amount of the peptides D-Arg-2'6'-Dmt-Lys-Phe-NH₂ or a pharmaceutically acceptable salt thereof; and
   (b) performing a revascularization procedure on the subject.
2. The method of claim 1, wherein the subject is administered the peptide prior to the revascularization procedure.
3. The method of paragraph 1, wherein the subject is administered the peptide after the revascularization procedure.
4. The method of paragraph 1, wherein the subject is administered the peptide during and after the revascularization procedure.
5. The method of paragraph 1, wherein the subject is administered the peptide continuously before, during, and after the revascularization procedure.
6. The method of paragraph 5, wherein the subject is administered the peptide for at least 3 hours after the revascularization procedure.
7. The method of paragraph 5, wherein the subject is administered the peptide starting at about 1 hour before the revascularization procedure.
8. The method of paragraph 5, wherein the subject is administered the peptide starting at about 30 minutes before the revascularization procedure.
9. The method of paragraph 1, wherein the subject is suffering from a myocardial infarction.
10. The method of paragraph 1, wherein the subject is suffering from a ST elevation myocardial infarction, or a non-ST elevation myocardial infarction.
11. The method of paragraph 1, wherein the subject is in need of angioplasty.
12. The method of paragraph 1, wherein the revascularization procedure is selected from the group consisting of: balloon angioplasty; insertion of a stent; percutaneous transluminal coronary angioplasty; or directional coronary atherectomy.
13. The method of paragraph 1, wherein the revascularization procedure is removal of the occlusion.
14. The method of paragraph 1, wherein the revascularization procedure is administration of one or more thrombolytic agents.
15. The method of paragraph 14, wherein the one or more thrombolytic agents are selected from the group consisting of: tissue plasminogen activator; urokinase; prourokinase; streptokinase; acylated form of plasminogen; acylated form of plasmin; and acylated streptokinase-plasminogen complex.
16. The method of paragraph 1, where in the vessel occlusion is a cardiac vessel occlusion.
17. The method of paragraph 1. wherein the vessel occlusion is an intracranial vessel occlusion.
18. The method of paragraph 1, wherein the vessel occlusion is a renal vessel occlusion.
19. The method of paragraph 1, wherein the vessel occlusion is selected from the group consisting of: deep venous thrombosis; peripheral thrombosis; embolic thrombosis; hepatic vein thrombosis; sinus thrombosis; venous thrombosis; an occluded arterio-venal shunt; and an occluded catheter device.
20. The method of paragraph 1, wherein the levels of one or more of CK-MB, troponin, N-terminal pro-brain natriuretic peptide (NT-proBNP), glucose, and estimated glomerular filtration rate (eGFR) are reduced in a subject administered the peptide relative to a comparable subject undergoing a revascularization procedure, but not administered the peptide.
21. The method of paragraph 1, wherein the incidence of re-infarction, congestive heart failure, repeat revascularization procedure, renal failure or death in the hospital following the revascularization procedure are reduced in a subject administered the peptide relative to a comparable subject undergoing a revascularization procedure, but not administered the peptide.
22. The method of paragraph 1, wherein the incidence of Major Adverse Cardiovascular Events, death, cardiac death, or the development of congestive heart failure within 6 months following the revascularization procedure are reduced in a subject administered the peptide relative to a comparable subject undergoing a revascularization procedure, but not administered the peptide.

### BRIEF DESCRIPTION OF THE FIGURES

FIG. 1 is an illustration of the study design for animals used in the examples.
FiGs. 2A and 2B present data showing infarct size for rabbits with a sham treatment (ligature applied, but not tightened). FIG. 2A is a photograph of heart slices and a computer-generated image highlighting infarct size of a sham rabbit treated with a placebo. FIG. 2B is a photograph of heart slices and a computer-generated image highlighting infarct size of a sham rabbit treated with peptide.
FIGs. 3A and 3B present data showing infarct size for two different control rabbits with induced cardiac ischemia and treated with a placebo. Each figure shows a photograph of heart slices and a computer-generated image highlighting infarct size.
FIGs. 4A, 4B, 4C, 4D, and 4E present data showing infarct size for five different rabbits with induced cardiac ischemia and treated with an illustrative aromatic-cationic peptide. Each figure shows a photograph of heart slices and a computer-generated image highlighting infarct size.
[**0028**] FIG. 5 is a chart showing the ratio of infracted area to left ventricular area for control and test groups of rabbits.
[**0029**] FIG. 6 is a chart showing the ratio of infracted area to area of risk for each of the control and test groups of rabbits.

### DETAILED DESCRIPTION

It is to be appreciated that certain aspects, modes, embodiment, variations and features of the invention are described below in various levels of detail in order to provide a substantial understanding of the present invention.

The definitions of certain terms as used in this specification are provided below. Unless defined otherwise, all technical and scientific terms used herein generally have the same meaning as commonly understood by one of ordinary skill in the art to which this invention belongs.

As used in this specification and the appended claims, the singular forms "a," "an" and "they" include plural referents unless the content clearly dictates otherwise. For example, reference to "a cell" includes a combination of two or more cells, and the like.

As used herein, the "administration" of an agent, drug, or peptide to a subject includes any route of introducing or delivering to a subject a compound to perform its intended function. Administration can be carried out by any suitable route, including orally, intranasally, parenterally (intravenously, intramuscularly, intraperitoneally, or subcutaneously), or topically. Administration includes self-administration and the administration by another.

As used herein, the term "amino acid" includes naturally-occurring amino acids and synthetic amino acids, as well as amino acid analogs and amino acid mimetics that function in a manner similar to the naturally-occurring amino acids. Naturally-occurring amino acids are those encoded by the genetic code, as well as those amino acids that are later modified, *e.g..* hydroxyproline, y-carboxyglutamate, and O-phosphoserine. Amino acid analogs refers to compounds that have the same basic chemical structure as a naturally-occurring amino acid, *i.e.,* an α-carbon that is bound to a hydrogen, a carboxyl group, an amino group, and an R group, *e.g..* homoserine, norleucine, methionine sulfoxide, methionine methyl sulfonium. Such analogs have modified R groups (*e.g.,* norleucine) or modified peptide backbones, but retain the same basic chemical structure as a naturally-occurring amino acid. Amino acid mimetics refers to chemical compounds that have a structure that is different from the general chemical structure of an amino acid, but that functions in a manner similar to a naturally-occurring amino acid. Amino acids can be referred to herein by either their commonly known three letter symbols or by the one-letter symbols recommended by the IUPAC-IUB Biochemical Nomenclature Commission.

As used herein, the term "effective amount" refers to a quantity sufficient to achieve a desired therapeutic and/or prophylactic effect, *e.g.,* an amount which results in the prevention of, or a decrease in, cardiac ischemia-reperfusion injury or one or more symptoms associated with cardiac ischemia-reperfusion injury. In the context of therapeutic or prophylactic applications, the amount of a composition administered to the subject will depend on the type and severity of the disease and on the characteristics of the individual, such as general health, age, sex, body weight and tolerance to drugs, It will also depend on the degree, severity and type of disease. The skilled artisan will be able to determine appropriate dosages depending on these and other factors. The compositions can also be administered in combination with one or more additional therapeutic compounds. In the methods described herein, the aromatic-cationic peptides may be administered to a subject having one or more signs or symptoms of vessel occlusion. In other embodiments, the mammal has one or more signs or symptoms of myocardial infarction, such as chest pain described as a pressure sensation, fullness, or squeezing in the mid portion of the thorax; radiation of chest pain into the jaw or teeth, shoulder, arm, and/or back; dyspnea or shortness of breath; epigastric discomfort with or without nausea and vomiting; and diaphoresis or sweating. For example, a "therapeutically effective amount" of the aromatic-cationic peptides is meant levels in which the physiological effects of a vessel occlusion injury and revascularization are, at a minimum, ameliorated.

As used herein the term "ischemia reperfusion injury"' refers to the damage caused first by restriction of the blood supply to a tissue followed by a resupply of blood and the attendant generation of free radicals. Ischemia is a decrease in the blood supply to the tissue and is followed by reperfusion, a sudden perfusion of oxygen into the deprived tissue.

An "isolated" or "purified" polypeptide or peptide is substantially free of cellular material or other contaminating polypeptides from the cell or tissue source from which the agent is derived, or substantially free from chemical precursors or other chemicals when chemically synthesized. For example, an isolated aromatic-cationic peptide would be free of materials that would interfere with diagnostic or therapeutic uses of the agent. Such interfering materials may include enzymes, hormones and other proteinaceous and nonproteinaceous solutes.

As used herein, the terms "polypeptide," "peptide," and "protein" are used interchangeably herein to mean a polymers comprising two or more amino acids joined to each other by peptide bonds or modified peptide bonds, *i.e.,* peptide isosteres. Polypeptide refers to both short chains, commonly referred to as peptides, glycopeptides or oligomers, and to longer chains, generally referred to as proteins. Polypeptides may contain amino acids other than the 20 gene-encoded amino acids. Polypeptides include amino acid sequences modified either by natural processes, such as post-translational processing, or by chemical modification techniques that are weal known in the art.

As used herein, the terms "treating" or "treatment" or "alleviation" refers to both therapeutic treatment and prophylactic or preventative measures, wherein the object is to prevent or slow down (lessen) the targeted pathologic condition or disorder. A subject is successfully "treated" for vessel occlusion injury if, after receiving a therapeutic amount of the aromatic-cationic peptides according to the methods described herein, the subject shows observable and/or measurable reduction in or absence of one or more signs and symptoms of vessel occlusion injury. It is also to be appreciated that the various modes of treatment or prevention of medical conditions as described are intended to mean "substantial," which includes total but also less than total treatment or prevention, and wherein some biologically or medically relevant result is achieved.

As used herein, "prevention'" or "preventing'" of a disorder or condition refers to a compound that reduces the occurrence of the disorder or condition in the treated sample relative to an untreated control sample, or delays the onset or reduces the severity of one or more symptoms of the disorder or condition relative to the untreated control sample. As used herein, preventing ischemia-reperfusion injury includes preventing oxidative damage or preventing mitochondrial permeability transitioning, thereby preventing or ameliorating the harmful effects of the loss and subsequent restoration of blood flow to an effected organ, Preventing does not mean that a subject never develops the condition later in life - only that the probability of occurrence is reduced.

### Methods of Prevention or Treatment

The present technology relates to the treatment or prevention of vessel occlusion injury by administration of certain aromatic-cationic peptides in conjunction with a revascularization procedure. Also provided is a method for the treatment or prevention of cardiac ischemia-reperfusion injury. Also provided is a method of treating a myocardial infarction in a subject to prevent injury to the heart upon reperfusion.

In another embodiment, the subject is administered the peptide during and after the ischemia. In yet another embodiment, the subject is administered the peptide continuously before, during, and after ischemia,. In another embodiment, the subject is administered the peptide during and after the reperfusion. In yet another embodiment, the subject is administered the peptide continuously before, during, and after reperfusion, In one embodiment, the subject is administered the peptide as a continuous IV infusion from immediately prior to reperfusion for about 1 to 3 hours after reperfusion. Thereafter, the subject may be administered the peptide chronically by any route of administration.

In one embodiment, the subject is administered the peptide prior to a revascularization procedure. In another embodiment, the subject is administered the peptide after the revascularization procedure. In another embodiment, the subject is administered the peptide during and after the revascularization procedure. In yet another embodiment, the subject is administered the peptide continuously before, during, and after the revascularization procedure. In another embodiment, the subject is administered the peptide regularly (*i.e.,* chronically) following an AMI and/or a revascularization procedure. In one embodiment, the subject is administered for at least one week, at least one month or at least one year after the revascularization procedure.

Various methods can be used to evaluate the efficacy of the aromatic cationic peptides, including cadiac MRI, Biomarkers include Troponin, creatine kinase (CK), lactate dehydrogenase (LDH), and glycogen phosphorylase isoenzyme BB. Troponin is the most sensitive and specific marker for myocardial damage. Its peak serum concentration is at 12 hours after damage and has delayed release for up to 7 days. Commercial kits for troponin I and T are available. Creatine kinase (CK) is a specific cardiac marker when skeletal muscle damage is not present. It peaks at 10-14 hours after damage and returns to normal 2-3 days.

The aromatic-cationic peptides are water-soluble and highly polar. Despite these properties, the peptides can readily penetrate cell membranes, The aromatic-cationic peptides typically include a minimum of three amino acids or a minimum of four amino acids, covalently joined by peptide bonds. The maximum number of amino acids present in the aromatic-cationic peptides is about twenty amino acids covalently joined by peptide bonds. Suitably, the maximum number of amino acids is about twelve, more preferably about nine, and most preferably about six.

The amino acids of the aromatic-cationic peptides can be any amino acid. As used herein, the term "amino acid" is used to refer to any organic molecule that contains at least one amino group and at least one carboxyl groups, Typically, at least one amino group is at the a position relative to a carboxyl group. The amino acids may be naturally occurring. Naturally occurring amino acids include, for example, the twenty most common levorotatory (L) amino acids normally found in mammalian proteins, *i*.*e*., alanine (Ala), arginine (Arg), asparagine (Asn), aspartic acid (Asp), cysteine (Cys), glutamine (Gln), glutamic acid (Glu), glycine (Gly), histidine (His), isoleucine (Ile), leucine (Leu), lysine (Lys), methionine (Met), phenylalanine (Phe), proline (Pro), serine (Ser), threonine (Thr), tryptophan, (Trp), tyrosine (Tyr), and valine (Val). Other naturally occurring amino acids include, for example, amino acids that are synthesized in metabolic processes not associated with protein synthesis. For example, the amino acids ornithine; and citrulline are synthesized in mammalian metabolism during the production of urea. Another example of a naturally occurring amino acid includes hydroxyproline (Hyp).

The peptides optionally contain one or more non-naturally occurring amino acids. Optimally, the peptide has no amino acids that are naturally occurring. The not-naturally occurring amino acids may be levorotary (L-), dextrorotatory (D-), or mixtures thereof. Non-naturally occurring amino acids are those amino acids that typically are not synthesized in normal metabolic processes in living organisms, and do not naturally occur in proteins. In addition, the non-naturally occurring amino acids suitably are also not recognized by common proteases. The non-naturally occurring amino acid can be present at any position in the peptide. For example, the non-naturally occurring amino acid can be at the N-termmus, the C-terminus, or at any position between the N-terminus and the C-terminus.

The non-natural amino acids may, for example, comprise alkyl, aryl, or alkylaryl groups not found in natural amino acids, Some examples of non-natural alkyl amino acids include α-aminobutyric acid, β-aminobutyric acid, γ-aminobutyric acid, δ-aminovaleric acid, and ε-aminocaproic acid. Some examples of non-natural aryl amino acids include ortho-, meta, and para-aminobenzoic acid, Some examples of non-natural alkylaryl amino acids include ortho-, meta-, and para-aminophenylacetic acid, and γ-phenyl-β-aminobutyric acid. Non-naturally occurring amino acids include derivatives of naturally occurring amino acids. The derivatives of naturally occurring amino acids may, for example, include the addition of one or more chemical groups to the naturally occurring amino acid.

For example, one or more chemical groups can be added to one or more of the 2', 3', 4', 5', or 6' position of the aromatic ring of a phenylalanine or tyrosine residue, or the 4', 5', 6', or 7' position of the benzo ring of a tryptophan residue. The group can be any chemical group that can be added to an aromatic ring. Some examples of such groups include branched or unbranched C₁-C₄ alkyl, such as methyl, ethyl, n-propyl, isopropyl, butyl, isobutyl, or t-butyl, C₃-C₄ alkyloxy (*i.e*., alkoxy), amino, C₁-C₄ alkylamino and C₁-C₄ dialkylamino (*e.g.,* methylamino, dimethylamino), nitro, hydroxyl, halo (*i*.*e*., fluoro, chloro, bromo, or iodo). Some specific examples of non-naturally occurring derivatives of naturally occurring amino acids include norvaline (Nva) and norleucine (Nle).

Another example of a modification of an amino acid in a peptide is the derivatization of a carboxyl group of an aspartic acid or a glutamic acid residue of the peptide. One example of derivatization is amidation with ammonia or with a primary or secondary amine, *e.g.* methylamine, ethylamine, dimethylamine or diethylamine. Another example of derivatization includes esterification with, for example, methyl or ethyl alcohol. Another such modification includes derivatization of an amino group of a lysine, arginine, or histidine residue. For example, such amino groups can be acylated. Some suitable acyl groups include, for example, a benzoyl group or an alkanoyl group comprising any of the C₁-C₄ alkyl groups mentioned above, such as an acetyl or propionyl group.

The non-naturally occurring Amino acids are suitably resistant, and/or insensitive, to common proteases. Examples of non-naturally occurring amino acids that are resistant or insensitive to proteases include the dextrorotatory (D-) form of any of the above-mentioned naturally occurring L-amino acids, as well as L- and/or D- non-naturally occurring amino acids. The D-amino acids do not normally occur in proteins, although they are found in certain peptide antibiotics that are synthesized by means other than the normal ribosomal protein synthetic machinery of the cell. As used herein, the D-amino acids are considered to be non-naturally occurring amino acids.

In order to minimize protease sensitivity, the peptides may have less than five, less than four, less than three, or less than two contiguous L-amino acids recognized by common proteases, irrespective of whether the amino acids are naturally or non-naturally occurring., Optimally, the peptide has only D-amino acids, and no L-amino acids. If the peptide contains protease sensitive sequences of amino acids, at least one of the amino acids is suitably a non-naturally-occurring D-amino acid, thereby conferring protease resistance. An example of a protease sensitive sequence includes two or more contiguous basic amino acids that are readily cleaved by common proteases, such as endopeptidases and trypsin. Examples of basic amino acids include arginine, lysine and histidine.

The aromatic-cationic peptides should have a minimum number of net positive charges at physiological pH in comparison to the total number of amino acid residues in the peptides. The minimum number of net positive charges at physiological pH will be referred to below as (pₘ). The total number of amino acid residues in the peptide will be referred to below as (r). The minimum number of net positive charges discussed below are all at physiological pH. The term "physiological pH**"** as used herein refers to the normal pH in the cells of the tissues and organs of the mammalian body. For instance, the physiological pH of a human is normally approximately 7.4, but normal physiological pH in mammals may be any pH from about 7.0 to about 7,8.

"Net charge" as used herein refers to the balance of the number of positive charges and the number of negative charges carried by the amino acids present in the peptide, In this specification, it is understood that net charges are measured at physiological pH. The naturally occurring amino acids that are positively charged at physiological pH include L-lysine, L-arginine, and L-histidine. The naturally occurring amino acids that are negatively charged at physiological pH include L-aspartic acid and L-glutamic acid.

Typically, a peptide has a positively charged N-terminal Amino group and a negatively charged C-terminal carboxyl group. The charges cancel each other out at physiological pH. As an example of calculating net charge, the peptide Tyr-Arg-Phe-Lys" Glu-His-Trp-D-Arg has one negatively charged amino acid (*i*.*e*., Glu) and four positively charged amino acids *(i.e.,* two Arg residues, one Lys, and one His). Therefore, the above peptide has a net positive charge of three.

In one embodiment, the aromatic-cationic peptides have a relationship between the minimum number of net positive charges at physiological pH (pₘ) and the total number of amino acid residues (r) wherein 3pₘ is the largest number that is less than or equal to r + 1. In this embodiment, the relationship between the minimum number of net positive charges (pₘ) and the total number of amino acid residues (r) is as follows:

**TABLE 1. Amino acid number and net positive charges (3pₘ ≤ p+1)**

| | | | | | | | | | | | | | | | | | | |
|---|---|---|---|---|---|---|---|---|---|---|---|---|---|---|---|---|---|---|
| **(r)** | 3 | 4 | 5 | 6 | 7 | 8 | 9 | 10 | 11 | 12 | 13 | 14 | 15 | 16 | 17 | 18 | 19 | 20 |
| **(Pₘ)** | 1 | 1 | 2 | 2 | 2 | 3 | 3 | 3 | 4 | 4 | 4 | 5 | 5 | 5 | 6 | 6 | 6 | 7 |

In another embodiment, the aromatic-cationic peptides have a relationship between the minimum number of net positive charges (pₘ) and the total number of amino acid residues (r) wherein 2pₘ is the largest number that is less than or equal to r + 1. In this embodiment, the relationship between the minimum number of net positive charges (pₘ) and the total number of amino acid residues (r) is as follows:

**TABLE 2. Amino acid number and net positive charges (2pₘ ≤ p+1)**

| | | | | | | | | | | | | | | | | | | |
|---|---|---|---|---|---|---|---|---|---|---|---|---|---|---|---|---|---|---|
| **(r)** | 3 | 4 | 5 | 6 | 7 | 8 | 9 | 10 | 11 | 12 | 13 | 14 | 15 | 16 | 17 | 18 | 19 | 20 |
| **(Pₘ)** | 2 | 2 | 3 | 3 | 4 | 4 | 5 | 5 | 6 | 6 | 7 | 7 | 8 | 8 | 9 | 9 | 10 | 10 |

In one embodiment, the minimum number of net positive charges (pₘ) and the total number of amino acid residues (r) are equal. In another embodiment, the peptides have three or four amino acid residues and a minimum of one net positive charge, suitably, a minimum of two net positive charges and more preferably a minimum of three net positive charges.

It is also important that the aromatic-cationic peptides have a minimum number of aromatic groups in comparison to the total number of net positive charges (pₜ). The minimum number of aromatic groups will be referred to below as (a). Naturally occurring amino acids that have an aromatic group include the amino acids histidine, tryptophan, tyrosine, and phenylalanine. For example, the hexapeptide Lys-Gln-Tyr-D-Arg-Phe-Trp has a net positive charge of two (contributed by the lysine and arginine residues) and three aromatic groups (contributed by tyrosine, phenylalanine and tryptophan, residues).

The aromatic-cationic peptides should also have a relationship between the minimum number of aromatic groups (a) and the total number of net positive charges at physiological pH (pₜ) wherein 3a is the largest number that is less than or equal to pₜ + 1, except that when pₜ is 1, a may also be 1. In this embodiment, the relationship between the minimum number of aromatic groups (a) and the total number of net positive charges (pt is as follows:

**TBALE 3. Aromatic groups and net posibive charges (3a ≤ pₜ+1 or a=pₜ=1)**

| | | | | | | | | | | | | | | | | | | | | |
|---|---|---|---|---|---|---|---|---|---|---|---|---|---|---|---|---|---|---|---|---|
| **(pₜ)** | 1 | 2 | 3 | 4 | 5 | 6 | 7 | 8 | 9 | 10 | 11 | 12 | 13 | 14 | 15 | 16 | 17 | 18 | 19 | 20 |
| **(a)** | 1 | 1 | 1 | 1 | 2 | 2 | 2 | 3 | 3 | 3 | 4 | 4 | 4 | 5 | 5 | 5 | 6 | 6 | 6 | 7 |

In another embodiment, the aromatic-cationic peptides have a relationship between the minimum number of aromatic groups (a) and the total number of net positive charges (pₜ) wherein 2a is the largest number that is less than or equal to pₜ + 1. In this embodiment, the relationship between the minimum number of aromatic amino acid residues (a) and the total number of net positive charges (pₜ) is as follows:

**TBALE 4. Aromatic groups and net posibive charges (2a ≤ pₜ+1 or a=pₜ=1)**

| | | | | | | | | | | | | | | | | | | | | |
|---|---|---|---|---|---|---|---|---|---|---|---|---|---|---|---|---|---|---|---|---|
| **(pₜ)** | 1 | 2 | 3 | 4 | 5 | 6 | 7 | 8 | 9 | 10 | 11 | 12 | 13 | 14 | 15 | 16 | 17 | 18 | 19 | 20 |
| **(a)** | 1 | 1 | 2 | 2 | 3 | 3 | 4 | 4 | 5 | 5 | 6 | 6 | 7 | 7 | 8 | 8 | 9 | 9 | 10 | 10 |

In another embodiment, the number of aromatic groups (a) and the total number of net positive charges (pₜ) are equal.

Carboxyl groups, especially the terminal carboxyl group of a C-terminal amino acid, are suitably amidated with, for example, ammonia to form the C-terminal amide. Alternatively, the terminal carboxyl group of the C-terminal amino acid may be amidated with any primary or secondary amine. The primary or secondary amine may, for example, be an alkyl, especially a branched or unbranched C₁-C₄ alkyl, or an aryl amine. Accordingly, the amino acid at the C-terminus of the peptide may be converted to an amido, N-methylamido, N-ethylamido, N,N-dimethylamido, N,N-diethylamido, N-methyl-N-ethylamido, N-phenylamido or N-phenyl-N-ethylamido group. The free carboxylate groups of the asparagine, glutamine, aspartic acid, and glutamic acid residues not occurring at the C-terminus of the aromatic-cationic peptides may also be amidated wherever they occur within the peptide. The amidation at these internal positions may be with ammonia or any of the primary or secondary amines described above.

In one embodiment, the aromatic-cationic peptide is a tripeptide having two net positive charges and at least one aromatic amino acid, In a particular embodiment, the aromatic-cationic peptide is a tripeptide having two net positive charges and two aromatic amino acids.

Aromatic-cationic peptides include, but are not limited to, the following peptide examples:

Lys-D-Arg-Tyr-NH2
Phe-D-Arg-His
D-Tyr-Trp-Lys-NH2
Trp-D-Lys-Tyr-Arg-NH2
Tyr-His-D-Gly-Met
Phe-Arg-D-His-Asp
Tyr-D-Arg-Phe-Lys-Glu-NH2
Met-Tyr-D-Lys-Phe-Arg

D-His-Glu-Lys-Tyr-D-Phe-Arg

Lys-D-Gln-Tyr-Arg-D-Phe-Trp-NH2

Phe-D-Arg-Lys-Trp-Tyr-D-Arg-His

Gly-D-Phe-Lys-Tyr-His-D-Arg-Tyr-NH2

Val-D-Lys-His-Tyr-D-Phe-Ser-Tyr-Arg-NH2

Trp-Lys-Phe-D-Asp-Arg-Tyr-D-His-Lys

Lys-Trp-D-Tyr-Arg-Asn-Phe-Tyr-D-His-NH2

Thr-Gly-Tyr-Arg-D-His-Phe-Trp-D-His-Lys

Asp-D-Trp-Lys-Tyr-D-His-Phe-Arg- D-Gly-Lys-NH2

D-His-Lys-Tyr- D-Phe-Glu- D-Asp- D-His- D-Lys-Arg-Trp-NH2

Ala-D-Phe-D-Arg-Tyr-Lys-D-Trp-His-D-Tyr-Gly-Phe

Tyr-D-His-Phe- D-Arg-Asp-Lys- D-Arg-His-Trp-D-His-Phe

Phe-Phe-D-Tyr-Arg-Glu-Asp-D-Lys-Arg-D-Arg-His-Phe-NH2

Phe-Try-Lys-D-Arg-Trp-His-D-Lys-D-Lys-Glu-Arg-D-Tyr-Thr

Tyr-Asp-D-Lys-Tyr-Phe- D-Lys- D-Arg-Phe-Pro-D-Tyr-His-Lys

Glu-Arg-D-Lys-Tyr- D-Val-Phe- D-His-Trp-Arg-D-Gly-Tyr-Arg-D-Met-NH2

Arg-D-Leu-D-Tyr-Phe-Lys-Glu- D-Lys-Arg-D-Trp-Lys- D-Phe-Tyr-D-Arg-Gly

D-Glu-Asp-Lys-D-Arg-D-His-Phe-Phe-D-Val-Tyr-Arg-Tyr-D-Tyr-Arg-His-Phe-
NH2

Asp-Arg-D-Phe-Cys-Phe-D-Arg-D-Lys-Tyr-Arg-D-Tyr-Trp-D-His-Tyr-D-Phe-Lys-
Phe

His-Tyr-D-Arg-Trp-Lys-Phe-D-Asp-Ala-Arg-Cys-D-Tyr-His-Phe-D-Lys-Tyr-His-
Ser-NH2

Gly-Ala-Lys-Phe-D-Lys-Glu-Arg-Tyr-His-D-Arg-D-Arg-Asp-Tyr-Trp-D-His-Trp-
His-D-Lys-Asp

Thr-Tyr-Arg-D-Lys-Trp-Tyr-Glu-Asp-D-Lys-D-Arg-His-Phe-D-Tyr-Gly-Val-Ile-D-
His-Arg-Tyr-Lys-NH2

In one embodiment, the peptides have mu-opioid receptor agonist activity (*i.e*., they activate the mu-opioid receptor). Mu-opioid activity can be assessed by radioligand binding to cloned mu-opioid receptors or by bioassays using the guinea pig ileum (Schiller et al, Eur J Med Chem, 35:895-901, 2000; Zhao et al, J Pharmacol Exp Ther, 307:947-954, 2003). Activation of the mu-opioid receptor typically elicits an analgesic effect. In certain instances, an aromatic-cationic peptide having mu-opioid receptor agonist activity is preferred. For example, during short-term treatment, such as in an acute disease or condition, it may be beneficial to use an aromatic-cationic peptide that activates the mu-opioid receptor. Such acute diseases and conditions are often associated with moderate or severe pain. In these instances, the analgesic effect of the aromatic-cationic peptide may be beneficial in the treatment regimen of the human patient or other mammal. An aromatic-cationic peptide which does not activate the mu-opioid receptor, however, may also be used with or without an analgesic, according to clinical requirements.

Alternatively, in other instances, an aromatic-cationic peptide that does not have mu-opioid receptor agonist activity is preferred. For example, during long-term treatment, such as in a chronic disease state or condition, the use of an aromatic-cationic peptide that activates the mu-opioid receptor may be contraindicated. In these instances, the potentially adverse or addictive effects of the aromatic-cationic peptide may preclude the use of an aromatic-cationic peptide that activates the mu-opioid receptor in the treatment regimen of a human patient or other mammal. Potential adverse effects may include sedation, constipation and respiratory depression. In such instances an aromatic-cationic peptide that does not activate the mu-opioid receptor may be an appropriate treatment.

Peptides which have mu-opioid receptor agonist activity are typically those peptides, which have a tyrosine residue or a tyrosine derivative at the N-terminus (*i.e*., the first amino acid position). Suitable derivatives of tyrosine include 2'-methyltyrosine (Mmt); 2',6'-dimethyltyrosine (2'6'-Dmt); 3',5'-dimethyltyrosine (3'5'Dmt); N,2',6'-trimethyltyrosine (Tmt); and 2'-hydroxy-6'-methyltryosine (Hmt).

In one embodiment, a peptide that has mu-opioid receptor agonist activity has the formula Tyr-D-Arg-Phe-Lys-NH₂. This peptide has a net positive charge of three, contributed by the amino acids tyrosine, arginine, and lysine and has two aromatic groups contributed by the amino acids phenylalanine and tyrosine. The tyrosine can be a modified derivative of tyrosine such as in 2',6'-dimethyltyrosine to produce the compound having the formula 2',6'-Dmt-D-Arg-Phe-Lys-NH₂. This peptide has a molecular weight of 640 and carries a net three positive charge at physiological pH. The peptide readily penetrates the plasma membrane of several mammalian cell types in an energy-independent manner (Zhao et al., J. Pharmacol Exp Ther., 304:425-432, 2003).

Peptides that do not have mu-opioid receptor agonist activity generally do not have a tyrosine residue or a derivative of tyrosine at the N-terminus (*i.e*., amino acid position 1). The amino acid at the N-terminus can be any naturally occurring or non-naturally occurring amino acid other than tyrosine. In one embodiment, the amino acid at the N-terminus is phenylalanine or its derivative. Exemplary derivatives of phenylalanine include 2'-methylphenylalanine (Mmp), 2',6'-dimethylphenylalanine (2',6'-Dmp), N,2',6'-trimethylphenylalanine (Tmp), and 2'-hydroxy-6-methylphenylalanine (Hmp).

An example of an aromatic-cationic peptide that does not have mu-opioid receptor agonist activity has the formula Phe-D-Arg-Phe-Lys-NH₂. Alternatively, the N-terminal phenylalanine can be a derivative of phenylalanine such as 2',6'-dimethylphenylalanine (2'6'-Dmp). In one embodiment, a peptide with 2',6'-dimethylphenylalanine at amino acid position 1 has the formula 2',6'-Dmp-D-Arg-Phe-Lys-NH₂. In one embodiment, the amino acid sequence is rearranged such that Dmt is not at the N-terminus. An example of such an aromatic-cationic peptide that does not have mu-opioid receptor agonist activity has the formula D-Arg-2'6'-Dmt-Lys-Phe-NH₂.

The peptides mentioned herein and their derivatives can further include functional analogs. A peptide is considered a functional analog if the analog has the same function as the stated peptide. The analog may, for example, be a substitution variant of a peptide, wherein one or more amino acids are substituted by another amino acid. Suitable substitution variants of the peptides include conservative amino acid substitutions. Amino acids may be grouped according to their physicochemical characteristics as follows:
(a) Non-polar amino acids: Ala(A) Ser(S) Thr(T) Pro(P) Gly(G) Cys (C);
(b) Acidic amino acids: Asn(N) Asp(D) Glu(E) Gln(Q);
(c) Basic amino acids: His(H) Arg(R) Lys(K);
(d) Hydrophobic amino acids: Met(M) Leu(L) Ile(I) Val(V); and
(e) Aromatic amino acids: Phe(F) Tyr(Y) Trp(W) His (H).

Substitutions of an amino acid in a peptide by another amino acid in the same group is referred to as a conservative substitution and may preserve the physicochemical characteristics of the original peptide. In contrast, substitutions of an amino acid in a peptide by another amino acid in a different group is generally more likely to alter the characteristics of the original peptide.

In some embodiments, one or more naturally occurring amino acids in the aromatic-cationic peptides are substituted with amino acid analogs. Examples of peptides include, but are not limited to, the aromatic-cationic peptides shown in Table 5.

**TABLE 5. Peptide Analogs with Mu-Opioid Activity**

| **Amino Acid Position 1** | **Amino Acid Position 2** | **Amino Acid Position 3** | **Amino Acid Position 4** | **C-Terminal Modification** |
|---|---|---|---|---|
| Tyr | D-Arg | Phe | Lys | NH₂ |
| Tyr | D-Arg | Phe | Orn | NH₂ |
| Tyr | D-Arg | Phe | Dab | NH₂ |
| Tyr | D-Arg | Phe | Dap | NH₂ |
| 2'6'Dmt | D-Arg | Phe | Lys | NH₂ |
| 2'6'Dmt | D-Arg | Phe | Lys-NH(CH₂)₂-NH-dns | NH₂ |
| 2'6'Dmt | D-Arg | Phe | Lys-NH(CH₂)₂-NH-atn | NH₂ |
| 2'6'Dmt | D-Arg | Phe | dnsLys | NH₂ |
| 2'6'Dmt | D-Cit | Phe | Lys | NH₂ |
| 2'6'Dmt | D-Cit | Phe | Ahp | NH₂ |
| 2'6'Dmt | D-Arg | Phe | Orn | NH₂ |
| 2'6'Dmt | D-Arg | Phe | Dab | NH₂ |
| 2'6'Dmt | D-Arg | Phe | Dap | NH₂ |
| 2'6'Dmt | D-Arg | Phe | Ahp(2-aminoheptanoic acid) | NH₂ |
| Bio-2'6'Dmt | D-Arg | Phe | Lys | NH₂ |
| 3'5'Dmt | D-Arg | Phe | Lys | NH₂ |
| 3'5'Dmt | D-Arg | Phe | Orn | NH₂ |
| 3'5'Dmt | D-Arg | Phe | Dab | NH₂ |
| 3'5'Dmt | D-Arg | Phe | Dap | NH₂ |
| Tyr | D-Arg | Tyr | Lys | NH₂ |
| Tyr | D-Arg | Tyr | Orn | NH₂ |
| Tyr | D-Arg | Tyr | Dab | NH₂ |
| Tyr | D-Arg | Tyr | Dap | NH₂ |
| 2'6'Dmt | D-Arg | Tvr | Lys | NH₂ |
| 2'6'Dmt | D-Arg | Tyr | Orn | NH₂ |
| 2'6'Dmt | D-Arg | Tyr | Dab | NH₂ |
| 2'6'Dmt | D-Arg | Tyr | Dap | NH₂ |
| 2'6'Dmt | D-Arg | 2'6'Dmt | Lvs | NH₂ |
| 2'6'Dmt | D-Arg | 2'6'Dmt | Orn | NH₂ |
| 2'6'Dmt | D-Arg | 2'6'Dart | Dab | NH₂ |
| 2'6'Dmt | D-Arg | 2'6'Dmt | Dap | NH₂ |
| 3'5'Dmt | D-Arg | 3'5'Dmt | Arg | NH₂ |
| 3'5'Dmt | D-Arg | 3'5'Dmt | Lys | NH₂ |
| 3'5'Dmt | D-Arg | 3'5'Dmt | Orn | NH₂ |
| 3'5'Dmt | D-Arg | 3'5'Dmt | Dab | NH₂ |
| Tyr | D-Lys | Phe | Dap | NH₂ |
| Tyr | D-Lys | Phe | Arg | NH₂ |
| Tyr | D-Lys | Phe | Lys | NH₂ |
| Tyr | D-Lys | Phe | Orn | NH₂ |
| 2'6'Dmt | D-Lys | Phe | Dab | NH₂ |
| 2'6'Dmt | D-Lys | Phe | Dap | NH₂ |
| 2'6'Dmt | D-Lys | Phe | Arg | NH₂ |
| 2'6'Dmt | D-Lys | Phe | Lys | NH₂ |
| 3'5'Dmt | D-Lys | Phe | Orn | NH₂ |
| 3'5'Dmt | D-Lys | Phe | Dab | NH₂ |
| 3'5'Dmt | D-Lys | Phe | Dap | NH₂ |
| 3'5'Dmt | D-Lys | Phe | Arg | NH₂ |
| Tyr | D-Lys | Tyr | Lys | NH₂ |
| Tyr | D-Lys | Tyr | Orn | NH₂ |
| Tyr | D-Lys | Tyr | Dab | NH₂ |
| Tyr | D-Lys | Tyr | Dap | NH₂ |
| 2'6'Dmt | D-Lvs | Tyr | Lys | NH₂ |
| 2'6'Dmt | D-Lys | Tyr | Orn | NH₂ |
| 2'6'Dmt | D-Lys | Tyr | Dab | NH₂ |
| 2'6'Dmt | D-Lys | Tyr | Dap | NH₂ |
| 2'6'Dmt | D-Lys | 2'6'Dmt | Lys | NH₂ |
| 2'6'Dmt | D-Lys | 2'6'Dmt | Orn | NH₂ |
| 2'6'Dmt | D-Lys | 2'6'Dmt | Dab | NH₂ |
| 2'6'Dmt | D-Lys | 2'6'Dmt | Dap | NH₂ |
| 2'6'Dmt | D-Arg | Phe | dnsDap | NH₂ |
| 2'6'Dmt | D-Arg | Phe | atnDap | NH₂ |
| 3'5'Dmt | D-Lys | 3'5'Dmt | Lys | NH₂ |
| 3'5'Dmt | D-Lys | 3'5'Dmt | Orn | NH₂ |
| 3'5'Dmt | D-Lys | 3'5'Dmt | Dab | NH₂ |
| 3'5'Dmt | D-Lys | 3'5'Dmt | Dap | NH₂ |
| Tyr | D-Lys | Phe | Arg | NH₂ |
| Tyr | D-Orn | Phe | Arg | NH₂ |
| Tyr | D-Dab | Phe | Arg | NH₂ |
| Tyr | D-Dap | Phe | Arg | NH₂ |
| 2'6'Dmt | D-Arg | Phe | Arg | NH₂ |
| 2'6'Dmt | D-Lys | Phe | Arg | NH₂ |
| 2'6'Dmt | D-Orn | Phe | Arg | NH₂ |
| 2'6'Dmt | D-Dab | Phe | Arg | NH₂ |
| 3'5'Dmt | D-Dap | Phe | Arg | NH₂ |
| 3'5'Dmt | D-Arg | Phe | Arg | NH₂ |
| 3'5'Dmt | D-Lys | Phe | Arg | NH₂ |
| 3'5'Dmt | D-Orn | Phe | Arg | NH₂ |
| Tyr | D-Lys | Tyr | Arg | NH₂ |
| Tyr | D-Orn | Tyr | Arg | NH₂ |
| Tyr | D-Dab | Tyr | Arg | NH₂ |
| Tyr | D-Dap | Tyr | Arg | NH₂ |
| 2'6'Dmt | D-Arg | 2'6'Dmt | Arg | NH₂ |
| 2'6'Dmt | D-Lys | 2'6'Dmt | Arg | NH₂ |
| 2'6'Dmt | D-Orn | 2'6'Dmt | Arg | NH₂ |
| 2'6'Dmt | D-Dab | 2'6'Dmt | Arg | NH₂ |
| 3'5'Dmt | D-Dap | 3'5'Dmt | Arg | NH₂ |
| 3'5'Dmt | D-Arg | 3'5'Dmt | Arg | NH₂ |
| 3'5'Dmt | D-Lys | 3'5'Dmt | Arg | NH₂ |
| 3'5'Dmt | D-Orn | 3'5'Dmt | Arg | NH₂ |
| Mmt | D-Arg | Phe | Lys | NH₂ |
| Mmt | D-Arg | Phe | Orn | NH₂ |
| Mmt | D-Arg | Phe | Dab | NH₂ |
| Mmt | D-Arg | Phe | Dap | NH₂ |
| Tmt | D-Arg | Phe | Lys | NH₂ |
| Tmt | D-Arg | Phe | Orn | NH₂ |
| Tmt | D-Arg | Phe | Dab | NH₂ |
| Tmt | D-Arg | Phe | Dap | NH₂ |
| Hmt | D-Arg | Phe | Lys | NH₂ |
| Hmt | D-Arg | Phe | Orn | NH₂ |
| Hmt | D-Arg | Phe | Dab | NH₂ |
| Hmt | D-Arg | Phe | Dap | NH₂ |
| Mmt | D-Lys | Phe | Lys | NH₂ |
| Mmt | D-Lys | Phe | Orn | NH₂ |
| Mmt | D-Lys | Phe | Dab | NH₂ |
| Mmt | D-Lys | Phe | Dap | NH₂ |
| Mmt | D-Lys | Phe | Arg | NH₂ |
| Tmt | D-Lys | Phe | Lys | NH₂ |
| Tmt | D-Lys | Phe | Orn | NH₂ |
| Tmt | D-Lys | Phe | Dab | NH₂ |
| Tmt | D-Lys | Phe | Dap | NH₂ |
| Tmt | D-Lys | Phe | Arg | NH₂ |
| Hmt | D-Lys | Phe | Lys | NH₂ |
| Hmt | D-Lys | Phe | Orn | NH₂ |
| Hmt | D-Lys | Phe | Dab | NH₂ |
| Hmt | D-Lys | Phe | Dap | NH₂ |
| Hmt | D-Lys | Phe | Arg | NH₂ |
| Mmt | D-Lys | Phe | Arg | NH₂ |
| Mmt | D-Orn | Phe | Arg | NH₂ |
| Mmt | D-Dab | Phe | Arg | NH₂ |
| Mmt | D-Dap | Phe | Arg | NH₂ |
| Mmt | D-Arg | Phe | Arg | NH₂ |
| Tmt | D-Lys | Phe | Arg | NH₂ |
| Tmt | D-Orn | Phe | Arg | NH₂ |
| Tmt | D-Dab | Phe | Arg | NH₂ |
| Tmt | D-Dap | Phe | Arg | NH₂ |
| Tmt | D-Arg | Phe | Arg | NH₂ |
| Hmt | D-Lys | Phe | Arg | NH₂ |
| Hmt | D-Orn | Phe | Arg | NH₂ |
| Hmt | D-Dab | Phe | Arg | NH₂ |
| Hmt | D-Dap | Phe | Arg | NH₂ |
| Hmt | D-Arg | Phe | Arg | NH₂ |

| | | | | |
|---|---|---|---|---|
| Dab = diaminobutyric Dap = diaminopropionic acid Dmt = dimethyltyrosine Mmt = 2'-methyltyrosine Tmt = N, 2',6'-trimethyltyrosine Hmt = 2'-hydroxy,6'-methyltyrosine dnsDap = β-dansyl-L-α,β-diaminopropionic acid atnDap = β-anthraniloyl-L-α,β-diaminopropionic acid Bio = biotin | | | | |

Examples of analogs that do not activate mu-opioid receptors include, but are not limited to, the aromatic-cationic peptides shown in Table 6.

**TABLE 6. Peptide Analogs Lacking Mu-Opioid Activity**

| **Amino Add Position 1** | **Amino Acid Position 2** | **Amino Acid Position 3** | **Amino Add Position 4** | **C-Terminal Modification** |
|---|---|---|---|---|
| D-Arg | Dmt | Lys | Phe | NH₂ |
| D-Arg | Dmt | Phe | Lys | NH₂ |
| D-Arg | Phe | Lys | Dmt | NH₂ |
| D-Arg | Phe | Dmt | Lys | NH₂ |
| D-Arg | Lys | Dmt | Phe | NH₂ |
| D-Arg | Lys | Phe | Dmt | NH₂ |
| Phe | Lys | Dmt | D-Arg | NH₂ |
| Phe | Lys | D-Arg | Dmt | NH₂ |
| Phe | D-Arg | Phe | Lys | NH₂ |
| Phe | D-Arg | Dmt | Lys | NH₂ |
| Phe | D-Arg | Lys | Dmt | NH₂ |
| Phe | Dmt | D-Arg | Lys | NH₂ |
| Phe | Dmt | Lys | D-Arg | NH₂ |
| Lys | Phe | D-Arg | Dmt | NH₂ |
| Lys | Phe | Dmt | D-Arg | NH₂ |
| Lys | Dmt | D-Arg | Phe | NH₂ |
| Lys | Dmt | Phe | D-Arg | NH₂ |
| Lys | D-Arg | Phe | Dmt | NH₂ |
| Lys | D-Arg | Dmt | Phe | NH₂ |
| D-Arg | Dmt | D-Arg | Phe | NH₂ |
| D-Arg | Dmt | D-Arg | Dmt | NH₂ |
| D-Arg | Dmt | D-Arg | Tyr | NH₂ |
| D-Arg | Dmt | D-Arg | Trp | NH₂ |
| Trp | D-Arg | Phe | Lys | NH₂ |
| Trp | D-Arg | Tyr | Lys | NH₂ |
| Trp | D-Arg | Trp | Lys | NH₂ |
| Trp | D-Arg | Dmt | Lys | NH₂ |
| D-Arg | Trp | Lys | Phe | NH₂ |
| D-Arg | Trp | Phe | Lys | NH₂ |
| D-Arg | Trp | Lys | Dmt | NH₂ |
| D-Arg | Trp | Dmt | Lys | NH₂ |
| D-Arg | Lys | Trp | Phe | NH₂ |
| D-Arg | Lys | Trp | Dmt | NH₂ |
| Cha | D-Arg | Phe | Lys | NH₂ |
| Ala | D-Arg | Phe | Lys | NH₂ |

| | | | | |
|---|---|---|---|---|
| Cha = cyclohexyl alanine | | | | |

The amino acids of the peptides shown in Table 5 and 6 may be in either the L- or the D- configuration.

The peptides may be synthesized by any of the methods well known in the art. Suitable methods for chemically synthesizing the protein include, for example, those described by Stuart and Young in Solid Phase Peptide Synthesis, Second Edition, Pierce Chemical Company (1984), and in Methods Enzymol., 289, Academic Press, Inc, New York (1997).

### Prophylactic and Therapeutic Uses of Aromatic-Cationic Peptides.

*General.* The aromatic-cationic peptides described herein are useful to prevent or treat disease. Specifically, the disclosure provides for both prophylactic and therapeutic methods of treating a subject at risk of (or susceptible to) vessel occlusion injury or ischemia-reperfusion injury. Accordingly, the present methods provide for the prevention and/or treatment of vessel occlusion injury or ischemia-reperfusion injury in a subject by administering an effective amount of an aromatic-cationic peptide to a subject in need thereof.

In various embodiments, suitable *in vitro* or *in vivo* assays are performed to determine the effect of a specific aromatic-cationic peptide-based therapeutic and whether its administration is indicated for treatment. In various embodiments, *in vitro* assays can be performed with representative animal models, to determine if a given aromatic-cationic peptide-based therapeutic exerts the desired effect in preventing or treating ischemia-reperfusion injury. Compounds for use in therapy can be tested in suitable animal model systems including, but not limited to rats, mice, chicken, pigs, cows, monkeys, rabbits, and the like, prior to testing in human subjects. Similarly, for *in vivo* testing, any of the animal model systems known in the art can be used prior to administration to human subjects.

*Prophylactic Methods.* In one aspect, the invention provides a method for preventing, in a subject, vessel occlusion injury by administering to the subject an aromatic-cationic peptide that prevents the initiation or progression of the condition. Subjects at risk for vessel occlusion injury can be identified by, *e.g*., any or a combination of diagnostic or prognostic assays as described herein. In prophylactic applications, pharmaceutical compositions or medicaments of aromatic-cationic peptides are administered to a subject susceptible to, or otherwise at risk of a disease or condition in an amount sufficient to eliminate or reduce the risk, lessen the severity, or delay the outset of the disease, including biochemical, histologic and/or behavioral symptoms of the disease, its complications and intermediate pathological phenotypes presenting during development of the disease. Administration of a prophylactic aromatic-cationic can occur prior to the manifestation of symptoms characteristic of the aberrancy, such that a disease or disorder is prevented or, alternatively, delayed in its progression. The appropriate compound can be determined based on screening assays described above.

*Therapeutic Methods*. Another aspect of the technology includes methods of treating vessel occlusion injury or ischemia-reperfusion injury in a subject for therapeutic purposes. In therapeutic applications, compositions or medicaments are administered to a subject suspected of, or already suffering from such a disease in an amount sufficient to cure, or at least partially arrest, the symptoms of the disease, including its complications and intermediate pathological phenotypes in development of the disease. As such, the invention provides methods of treating an individual afflicted with ischemia-reperfusion injury.

### Modes of Administration and Effective Dosages

Any method known to those in the art for contacting a cell, organ or tissue with a peptide may be employed. Suitable methods include *in vitro, ex vivo, or in vivo* methods. *In vivo* methods typically include the administration of an aromatic-cationic peptide, such as those described above, to a mammal, suitably a human. When used *in vivo* for therapy, the aromatic-cationic peptides are administered to the subject in effective amounts (i.e., amounts that have desired therapeutic effect). The dose and dosage regimen will depend upon the degree of the injury in the subject, the characteristics of the particular aromatic-cationic peptide used, *e.g*., its therapeutic index, the subject, and the subject's history.

The effective amount may be determined during pre-clinical trials and clinical trials by methods familiar to physicians and clinicians. An effective amount of a peptides useful in the methods may be administered to a mammal in need thereof by any of a number of well-known methods for administering pharmaceutical compounds. The peptide may be administered systemically or locally.

The peptide may be formulated as a pharmaceutically acceptable salt. The term "pharmaceutically acceptable salt" means a salt prepared from a base or an acid which is acceptable for administration to a patient, such as a mammal (*e.g*., salts having acceptable mammalian safety for a given dosage regime). However, it is understood that the salts are not required to be pharmaceutically acceptable salts, such as salts of intermediate compounds that are not intended for administration to a patient. Pharmaceutically acceptable salts can be derived from pharmaceutically acceptable inorganic or organic bases and from pharmaceutically acceptable inorganic or organic acids. In addition, when a peptide contains both a basic moiety, such as an amine, pyridine or imidazole, and an acidic moiety such as a carboxylic acid or tetrazole, zwitterions may be formed and are included within the term "salt" as used herein. Salts derived from pharmaceutically acceptable inorganic bases include ammonium, calcium, copper, ferric, ferrous, lithium, magnesium, manganic, manganous, potassium, sodium, and zinc salts, and the like. Salts derived from pharmaceutically acceptable organic bases include salts of primary, secondary and tertiary amines, including substituted amines, cyclic amines, naturally-occurring amines and the like, such as arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine and the like. Salts derived from pharmaceutically acceptable inorganic acids include salts of boric, carbonic, hydrohalic (hydrobromic, hydrochloric, hydrofluoric or hydroiodic), nitric, phosphoric, sulfamic and sulfuric acids. Salts derived from pharmaceutically acceptable organic acids include salts of aliphatic hydroxyl acids (*e*.*g*., citric, gluconic, glycolic, lactic, lactobionic, malic, and tartaric acids), aliphatic monocarboxylic acids (*e.g*., acetic, butyric, formic, propionic and trifluoroacetic acids), amino acids (*e.g*., aspartic and glutamic acids), aromatic carboxylic acids (*e.g.,* benzoic, p-chlorobenzoic, diphenylacetic, gentisic, hippuric, and triphenylacetic acids), aromatic hydroxyl acids (*e.g.,* o-hydroxybenzoic, p-hydroxybenzoic, 1-hydroxynaphthalene-2-carboxylic and 3-hydroxynaphthalene-2-carboxylic acids), ascorbic, dicarboxylic acids (*e.g*., fumaric, maleic, oxalic and succinic acids), glucoronic, mandelic, mucic, nicotinic, orotic, pamoic, pantothenic, sulfonic acids (*e.g.,* benzenesulfonic, camphosulfonic, edisylic, ethanesulfonic, isethionic, methanesulfonic, naphthalenesulfonic, naphthalene-1,5-disulfonic, naphthalene-2,6-disulfonic and p-toluenesulfonic acids), xinafoic acid, and the like.

The aromatic-cationic peptides described herein can be incorporated into pharmaceutical compositions for administration, singly or in combination, to a subject for the treatment or prevention of a disorder described herein. Such compositions typically include the active agent and a pharmaceutically acceptable carrier. As used herein the term "pharmaceutically acceptable carrier" includes saline, solvents, dispersion media, coatings, antibacterial and antifungal agents, isotonic and absorption delaying agents, and the like, compatible with pharmaceutical administration. Supplementary active compounds can also be incorporated into the compositions.

Pharmaceutical compositions are typically formulated to be compatible with its intended route of administration. Examples of routes of administration include parenteral (*e.g*., intravenous, intradermal, intraperitoneal or subcutaneous), oral, inhalation, transdermal (topical), intraocular, iontophoretic, and transmucosal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic. For convenience of the patient or treating physician, the dosing formulation can be provided in a kit containing all necessary equipment (*e.g*., vials of drug, vials of diluent, syringes and needles) for a treatment course (*e.g*., 7 days of treatment).

Pharmaceutical compositions suitable for injectable use can include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany, N.J.) or phosphate buffered saline (PBS). In all cases, a composition for parenteral administration must be sterile and should be fluid to the extent that easy syringability exists. It should be stable under the conditions of manufacture and storage and must be preserved against the contaminating action of microorganisms such as bacteria and fungi.

The aromatic-cationic peptide compositions can include a carrier, which can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thiomerasol, and the like. Glutathione and other antioxidants can be included to prevent oxidation. In many cases, it will be preferable to include isotonic agents, for example, sugars, polyalcohols such as mannitol, sorbitol, or sodium chloride in the composition. Prolonged absorption of the injectable compositions can be brought about by including in the composition an agent which delays absorption, for example, aluminum monostearate or gelatin.

Sterile injectable solutions can be prepared by incorporating the active compound in the required amount in an appropriate solvent with one or a combination of ingredients enumerated above, as required, followed by filtered sterilization. Generally, dispersions are prepared by incorporating the active compound into a sterile vehicle, which contains a basic dispersion medium and the required other ingredients from those enumerated above. In the case of sterile powders for the preparation of sterile injectable solutions, typical methods of preparation include vacuum drying and freeze drying, which can yield a powder of the active ingredient plus any additional desired ingredient from a previously sterile-filtered solution thereof.

Oral compositions generally include an inert diluent or an edible carrier. For the purpose of oral therapeutic administration, the active compound can be incorporated with excipients and used in the form of tablets, troches, or capsules, *e*.*g*., gelatin capsules. Oral compositions can also be prepared using a fluid carrier for use as a mouthwash. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds can be delivered in the form of an aerosol spray from a pressurized container or dispenser which contains a suitable propellant, *e.g.,* a gas such as carbon dioxide, or a nebulizer. Such methods include those described in U.S. Pat. No. 6,468,798.

Systemic administration of a therapeutic compound as described herein can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays. For transdermal administration, the active compounds are formulated into ointments, salves, gels, or creams as generally known in the art. In one embodiment, transdermal administration may be performed my iontophoresis.

A therapeutic protein or peptide can be formulated in a carrier system. The carrier can be a colloidal system. The colloidal system can be a liposome, a phospholipid bilayer vehicle. In one embodiment, the therapeutic peptide is encapsulated in a liposome while maintaining peptide integrity. As one skilled in the art would appreciate, there are a variety of methods to prepare liposomes. (*See* Lichtenberg et al., Methods Biochem. Anal., 33:337-462 (1988); Anselem et al., Liposome Technology, CRC Press (1993)). Liposomal formulations can delay clearance and increase cellular uptake (*See* Reddy, Ann. Pharmacother., 34(7-8):915-923 (2000)). An active agent can also be loaded into a particle prepared from pharmaceutically acceptable ingredients including, but not limited to, soluble, insoluble, permeable, impermeable, biodegradable or gastroretentive polymers or liposomes. Such particles include, but are not limited to, nanoparticles, biodegradable nanoparticles, microparticles, biodegradable microparticles, nanospheres, biodegradable nanospheres, microspheres, biodegradable microspheres, capsules, emulsions, liposomes, micelles and viral vector systems.

The carrier can also be a polymer, *e.g.,* a biodegradable, biocompatible polymer matrix. In one embodiment, the therapeutic peptide can be embedded in the polymer matrix, while maintaining protein integrity. The polymer may be natural, such as polypeptides, proteins or polysaccharides, or synthetic, such as poly α-hydroxy acids. Examples include carriers made of, *e.g.,* collagen, fibronectin, elastin, cellulose acetate, cellulose nitrate, polysaccharide, fibrin, gelatin, and combinations thereof. In one embodiment, the polymer is poly-lactic acid (PLA) or copoly lactic/glycolic acid (PGLA). The polymeric matrices can be prepared and isolated in a variety of forms and sizes, including microspheres and nanospheres. Polymer formulations can lead to prolonged duration of therapeutic effect. (*See* Reddy, Ann. Pharmacother., 34(7-8):915-923 (2000)). A polymer formulation for human growth hormone (hGH) has been used in clinical trials. (*See* Kozarich and Rich, Chemical Biology, 2:548-552 (1998)).

Examples of polymer microsphere sustained release formulations are described in PCT publication WO 99/15154 (Tracy et al.), U.S. Pat. Nos. 5,674,534 and 5,716,644 (both to Zale et al.), PCT publication WO 96/40073 (Zale et al.), and PCT publication WO 00/38651 (Shah et al.). U.S. Pat. Nos. 5,674,534 and 5,716,644 and PCT publication WO 96/40073 describe a polymeric matrix containing particles of erythropoietin that are stabilized against aggregation with a salt.

In some embodiments, the therapeutic compounds are prepared with carriers that will protect the therapeutic compounds against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylacetic acid. Such formulations can be prepared using known techniques. The materials can also be obtained commercially, *e*.*g*., from Alza Corporation and Nova Pharmaceuticals, Inc. Liposomal suspensions (including liposomes targeted to specific cells with monoclonal antibodies to cell-specific antigens) can also be used as pharmaceutically acceptable carriers. These can be prepared according to methods known to those skilled in the art, for example, as described in U.S. Pat. No. 4,522,811.

The therapeutic compounds can also be formulated to enhance intracellular delivery. For example, liposomal delivery systems are known in the art, see, *e.g*., Chonn and Cullis, "Recent Advances in Liposome Drug Delivery Systems," Current Opinion in Biotechnology 6:698-708 (1995); Weiner, "Liposomes for Protein Delivery: Selecting Manufacture and Development Processes," Immunomethods, 4(3):201-9 (1994); and Gregoriadis, "Engineering Liposomes for Drug Delivery: Progress and Problems," Trends Biotechnol., 13(12):527-37 (1995). Mizguchi et al., Cancer Lett., 100:63-69 (1996), describes the use of fusogenic liposomes to deliver a protein to cells both *in vivo* and *in vitro.*

Dosage, toxicity and therapeutic efficacy of the therapeutic agents can be determined by standard pharmaceutical procedures in cell cultures or experimental animals, *e.g.,* for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds which exhibit high therapeutic indices are preferred. While compounds that exhibit toxic side effects may be used, care should be taken to design a delivery system that targets such compounds to the site of affected tissue in order to minimize potential damage to uninfected cells and, thereby, reduce side effects.

The data obtained from the cell culture assays and animal studies can be used in formulating a range of dosage for use in humans. The dosage of such compounds lies preferably within a range of circulating concentrations that include the ED50 with little or no toxicity. The dosage may vary within this range depending upon the dosage form employed and the route of administration utilized. For any compound used in the methods, the therapeutically effective dose can be estimated initially from cell culture assays. A dose can be formulated in animal models to achieve a circulating plasma concentration range that includes the IC50 (*i.e*., the concentration of the test compound which achieves a half-maximal inhibition of symptoms) as determined in cell culture. Such information can be used to more accurately determine useful doses in humans. Levels in plasma may be measured, for example, by high performance liquid chromatography.

Typically, an effective amount of the aromatic-cationic peptides, sufficient for achieving a therapeutic or prophylactic effect, range from about 0.000001 mg per kilogram body weight per day to about 10,000 mg per kilogram body weight per day. Preferably, the dosage ranges are from about 0.0001 mg per kilogram body weight per day to about 100 mg per kilogram body weight per day. For example dosages can be 1 mg/kg body weight or 10 mg/kg body weight every day, every two days or every three days or within the range of 1-10 mg/kg every week, every two weeks or every three weeks. In one embodiment, a single dosage of peptide ranges from 0.1-10,000 micrograms per kg body weight. In one embodiment, aromatic-cationic peptide concentrations in a carrier range from 0.2 to 2000 micrograms per delivered milliliter. An exemplary treatment regime entails administration once per day or once a week. In therapeutic applications, a relatively high dosage at relatively short intervals is sometimes required until progression of the disease is reduced or terminated, and preferably until the subject shows partial or complete amelioration of symptoms of disease. Thereafter, the patient can be administered a prophylactic regime.

In an exemplary embodiment, the subject is administered the peptide by intravenous infusion at about 0.001 to about 1 mg/kg/hr, *i.e*., about 0.005, about 0.01, about 0.025, about 0.05, about 0.10, about 0.25, or about 0.5 mg/kg/hour. The intravenous infusion may be started prior to or after reperfusion of the tissue. In some embodiments, the subject may receive in IV bolus injection prior to reperfusion of the tissue.

In some embodiments, a therapeutically effective amount of an aromatic-cationic peptide may be defined as a concentration of peptide at the target tissue of 10⁻¹² to 10⁻⁶ molar, *e*.*g*., approximately 10⁻⁷ molar. This concentration may be delivered by systemic doses of 0.01 to 100 mg/kg or equivalent dose by body surface area. The schedule of doses would be optimized to maintain the therapeutic concentration at the target tissue, most preferably by single daily or weekly administration, but also including continuous administration (*e.g*., parenteral infusion or transdermal application).

In some embodiments, the dosage of the aromatic-cationic peptide is provided at a "slow," "mid," or "high" dose level. In one embodiment, the low dose is provided from about 0.001 to about 0.5 mg/kg/h, suitably from about 0.01 to about 0.1 mg/kg/h. In one embodiment, the mid-dose is provided from about 0.1 to about 1.0 mg/kg/h, suitably from about 0.1 to about 0.5 mg/kg/h. In one embodiment, the high dose is provided from about 0.5 to about 10 mg/kg/h, suitably from about 0,5 to about 2 mg/kg/h.

The skilled artisan will appreciate that certain factors may influence the dosage and timing required to effectively treat a subject, including but not limited to, the severity of the disease or disorder, previous treatments, the general health and/or age of the subject, and other diseases present. Moreover, treatment of a subject with a therapeutically effective amount of the therapeutic compositions described herein can include a single treatment or a series of treatments.

The mammal treated in accordance present methods can be any mammal, including, for example, farm animals, such as sheep, pigs, cows, and horses; pet animals, such as dogs and cats; laboratory animals, such as rats, mice and rabbits, In a suitable embodiment, the mammal is a human.

### EXAMPLES

The present invention is further illustrated by the following example, which should not be construed as limiting in any way.

### Example 1. Effects of Aromatic-Cationic Peptides in Protecting Against Vessel Occlusion Injury In a Rabbit Model.

The effects of aromatic-cationic peptides in protecting against a vessel occlusion injury in a rabbit model were investigated. The myocardial protective effect of the peptide D-Arg-2'6'-Dmt-Lys-Phe-NH2 were demonstrated by this Example.

### Experimental Methods

New Zealand white rabbits were used in this study. The rabbits were males and > 10 weeks in age. Environmental controls in the animal rooms were set to maintain temperatures of 61° to 72°F and relative humidity between 30% and 70%. Room temperature and humidity were recorded hourly, and monitored daily. There were approximately 10 - 15 air exchanges per hour in the animal rooms. Photoperiod was 12-hr light/12-hr dark (*via* fluorescent lighting) with exceptions as necessary to accommodate dosing and data collection. Routine daily observations were performed. Harlan Teklad, Certified Diet (2030C), rabbit diet was provided approximately 180 grams per day from arrival to the facility. In addition, fresh fruits and vegetables were given to the rabbit 3 times a week.

The peptide D-Arg-2'6-Dmt-Lys-Phe-NH₂ (sterile lyophilized powder) was used as the test article. Dosing solutions were formulated at no more than 1 mg/ml, and were delivered *via* continuous infusion (IV) at a constant rate *(e.g.,* 50 µL/kg/min). Normal saline (0.9% NaCl) was used as a control.

The test/vehicle articles were given intravenously, under general anesthesia, in order to mimic the expected route of administration in the clinical setting of AMI and PTCA. Intravenous infusion was administered *via* a peripheral vein using a Kd Scientific infusion pump (Holliston, MA 01746) at a constant volume (*e.g*., 50 µL/kg/min).

The study followed a predetermined placebo and sham controlled design. In short, 10-20 healthy, acclimatized, male rabbits were assigned to one of three study arms (approximately 2-10 animals per group). Arm A (n = 10, CTRL/PLAC) includes animals treated with vehicle (vehicle; VEH, IV); Arm B (n =10, treated) includes animals treated with peptide; Arm C (n = 2, SHAM) includes sham-operated time-controls treated with vehicle (vehicle; VEH, IV) or peptide.

**Table 7. Study Design.**

| **Group** | **Study Group** | **Ischemia Time** | **Reperfusion Time** |
|---|---|---|---|
| A | CONTROL/PLACEBO | 30 Min (Last 20 Min. With Placebo) | 180 Min of Placebo |
| B | PEPTIDE | 30 Min (Last 20 Min. With Peptide) | 180 Min of Peptide |
| C | SHAM (FOR SURGERY WITHOUT ISCHEMIA) | 0 Min (Last 20 Min. With Placebo) | 180 Min of Placebo (Vehicle) or Peptide |

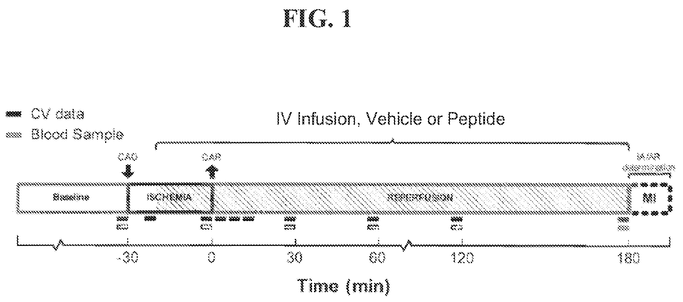

In all cases, treatments were started approximately 10 min after the onset of a 30 min ischemic insult (coronary occlusion) and continued for up to 3 h following reperfusion. In all cases, cardiovascular function was monitored both prior to and during ischemia, as well as for up to 180 min (3 h) post-reperfusion. The experiments were terminated 3 h post-reperfusion (end of study); irreversible myocardial injury (infarct size by histomorphometery) at this time-point was evaluated, and was the primary-end-point of the study. The study design is summarized in Table 7 and FIG. 1.

*Anesthesia*/*Surgical Preparation.* General anesthesia was induced intramuscularly (IM) with a ketamine (∼35 -50 mg/kg)/xylazine (∼5 -10 mg/kg) mixture. A venous catheter was placed in a peripheral vein (*e.g*., ear) for the administration of anesthetics. In order to preserve autonomic function, anesthesia was maintained with continuous infusions of propofol (∼8 - 30 mg/kg/hour) and ketamine (∼1.2 - 2.4 mg/kg/hr). A cuffed tracheal tube was placed *via* a tracheotomy (ventral midline incision) and used to mechanically ventilate the lungs with a 95% O₂/5% CO₂ mixture *via* a volume-cycled animal ventilator (∼40 breaths/minute with a tidal volume of ∼12.5 ml/kg) in order to sustain PaCO₂ values broadly within the physiological range.

Once a surgical plane of anesthesia was reached, either transthoracic or needle electrodes forming two standard ECG leads (*e.g*., lead II, aVF, V2) were placed. A cervical cut-down exposed a carotid artery, which was isolated, dissected free from the surrounding tissue and cannulated with a dual-sensor high-fidelity micromanometer catheter (Millar Instruments); the tip of this catheter was advanced into the left-ventricle (LV) retrogradely across the aortic valve, in order to simultaneously determine aortic (root, proximal transducer) and left-ventricular (distal transducer) pressures. The carotid cut-down also exposed the jugular vein, which was cannulated with a hollow injection catheter (for blood sampling). Finally, an additional venous catheter was placed in a peripheral vein (*e.g*., ear) for the administration of vehicle/test articles.

Subsequently, the animals were placed in right-lateral recumbence and the heart was exposed *via* a midline thoracotomy and a pericardiotomy. The heart was suspended on a pericardial cradle in order to expose the left circumflex (LCX) and the left-anterior descending (LAD) coronary arteries. Silk ligatures were loosely placed (using a taper-point needle) around the proximal LAD and if necessary, depending on each animal's coronary anatomy, around one or more branches of the LCX marginal coronary arteries. Tightening of these snares (*via* small pieces of polyethylene tubing) allowed rendering a portion of the left ventricular myocardium temporarily ischemic.

Once instrumentation was completed, hemodynamic stability and proper anesthesia depth were verified/ensured for at least 30 min. Subsequently, the animals were paralyzed with atracurium (∼0.1 to 0.2 mg/kg/hr IV) in order to facilitate hemodynamic/respiratory stability. Following atracurium administration, signs of autonomic hyperactivity and/or changes in BIS values were used to evaluate anesthesia depth and/or to up-titrate the intravenous anesthetics.

*Experimental Protocol*/*Cardiovascular Data Collection*. Immediately following surgical preparation, the animals were heparinized (100 units heparin/kg/h, IV bolus), and after hemodynamic stabilization (for approximately 30 min), baseline data were collected including venous blood for the evaluation of cardiac enzymes/biomarkers as well as of test-article concentrations.

Following hemodynamic stabilization and baseline measurements, the animals were subjected to an acute 30 min ischemic insult by tightening of the LAD/LCX coronary artery snares, Myocardial ischemia was visually confirmed by color (*i.e*., cyanotic) changes in distal distributions of the LAD/LCX and by the onset ofelectrocardiographic changes. Approximately after 10 min of ischemia, the animals received a continuous infusion of either vehicle (saline) or peptide; ischemia was continued for a additional 20 min (*i.e*., 30 min total) after the start of treatment. Subsequently (*i.e*., after 30 min of ischemia of which the last 20 min overlap with the treatment), the coronary snares were released and the previously ischemic myocardium was reperfused for up to 3 h. Treatment with either vehicle or peptide was continued throughout the reperfusion period. It should be noted that in sham-operated animals the vessel snares were manipulated at the time of ischemia/reperfusion onset, but were not either tightened or loosened.

Cardiovascular data collection occurred at 11 pre-determined time-points: post-instrumentation/stabilization (*i.e*., baseline), after 10 and 30 min of ischemia, as well as at 5, 15, 30, 60, 120, and 180 min post-reperfusion. Throughout the experiments, analog signals were digitally sampled (1000 Hz) and recorded continuously with a data acquisition system (IOX; EMKA Technologies), and the following parameters were determined at the above-mentioned time-points: (1) from bipolar transthoracic ECG (*e.g*., Lead II, aVF): rhythm (arrhythmia quantification/classification), RR, PQ, QRS, QT, QTc, short-term QT instability, and QT:TQ (restitution); (2) from solid-state manometer in aorta (Millar): arterial/aortic pressure (AoP); and (3) from solid-state manometer in the LV (Millar): left-ventricular pressures (ESP, EDP) and derived indices (dP/dtmax, dP/dtmin, Vmax, and tau). In addition, in order to determine/quantity the degree of irreversible myocardial injury (*i.e*., infarction) resulting from the I/R insult with and without peptide treatment, cardiac biomarkers as well as infarct area were evaluated.

*Blood Samples.* Venous (<3 mL) whole blood samples were collected for both pharmaco-kinetic (PK) analysis as well as for the evaluation of myocardial injury *via* cardiac biomarker analyses at six data-collection time-points: baseline, 30 min of ischemia, as well as 30, 60, 120 and 180 min post-reperfusion. In addition, three arterial (∼0.5 mL) whole blood samples were collected at baseline, 60 min of ischemia, as well as the 60 and 180 min post-reperfusion for the determination of blood-gases, the arterial samples were collected into blood gas syringes and used for the measurement of blood-gases *via* an 1-Stat analyzer/cartridges (CG4+).

*Histopathology*/*Histomorphometery.* At the completion of the protocol, irreversible myocardial injury (*i.e.*, infarction) resulting from the I/R insult was evaluated. In short, the coronary snares were retightened and Evan's blue dye (1 mL/kg; Sigma, St. Louis, MO) was injected intravenously to delineate the myocardial area-at-risk (AR) during ischemia. Approximately 5 min later, the heart was arrested (by an injection of potassium chloride into the left atrium), and freshly excised. The LV was sectioned perpendicular to its long axis (from apex to base) into 3 mm thick slices. Subsequently, the slices were incubated for 20 min in 2% triphenyl-tetrazolium-chloride (TTC) at 37°C and fixed in a 10% non-buffered formalin solution (NBF).

Following fixation, the infarct and at-risks areas were delineated/measured digitally. For such purpose, the thickness of each slice was measured with a digital micrometer and later photographed/scanned. All photographs were imported into an image analysis program (Image J; National Institutes of Health), and computer-assisted planometry was performed to determine the overall size of the infarct (1) and at-risk (AR) areas. For each slide, the AR (*i*.*e*., not stained blue) was expressed as a percentage of the LV area, and the infarct size (I, not stained tissue) was expressed as a percentage of the AR (I/AR). In all cases, quantitative histomorphometery was performed by personnel blinded to the treatment assignment/study-design.

*Animal Observations*. Data were acquired on the EMKA's IOX system using ECG Auto software for analysis (EMKA Technologies). Measurements for all physiological parameters were made manually or automatically from (digital) oscillograph tracings. The mean value from 60 s of data from each targeted time point was used (if possible); however, as mentioned above, signals/tracing was recorded continuously throughout the experiments, in order to allow (if needed) more fine/detailed temporal data analysis (*via* amendments). Additional calculations were performed using Microsoft Excel. Data is presented as means with standard errors.

### Results

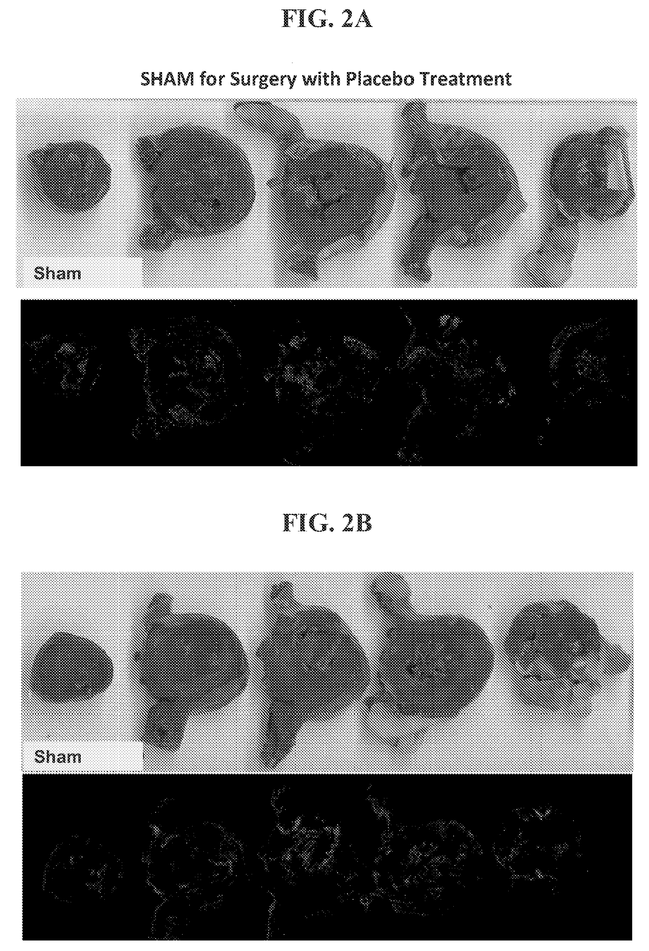

Infarct size from hearts exposed to 30 min ischemia and 3 h reperfusion is shown in FIGs. 2-6. FIGs. 2A and 2B present data showing infarct size for rabbits with a sham for surgery (ligature applied, but not tightened), with placebo or with peptide. The LV was sectioned perpendicular to its long axis (from apex to base) into 3 mm thick slices. FIG. 2A is a photograph of heart slices and a computer-generated image highlighting infarct size of a sham rabbit treated with a placebo. FIG. 2B is a photograph of heart slices and a computer-generated image highlighting infarct size of a sham rabbit treated with peptide.

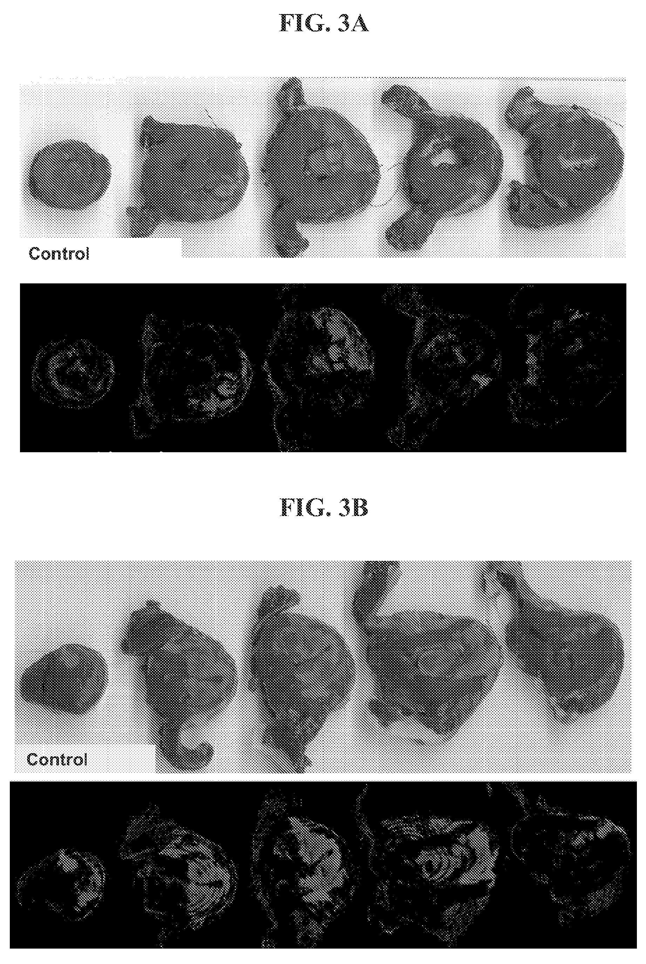

FIGs. 3A and 3B present data showing infarct size for two different control rabbits with induced cardiac ischemia and treated with a placebo. Each figure shows a photograph of heart slices and a computer-generated image highlighting infarct size.

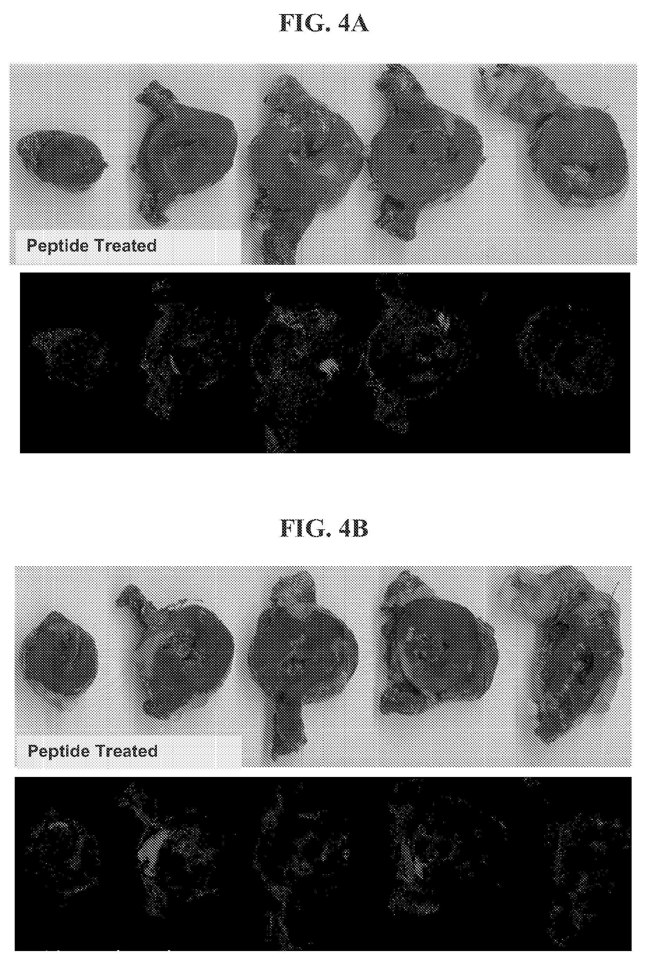

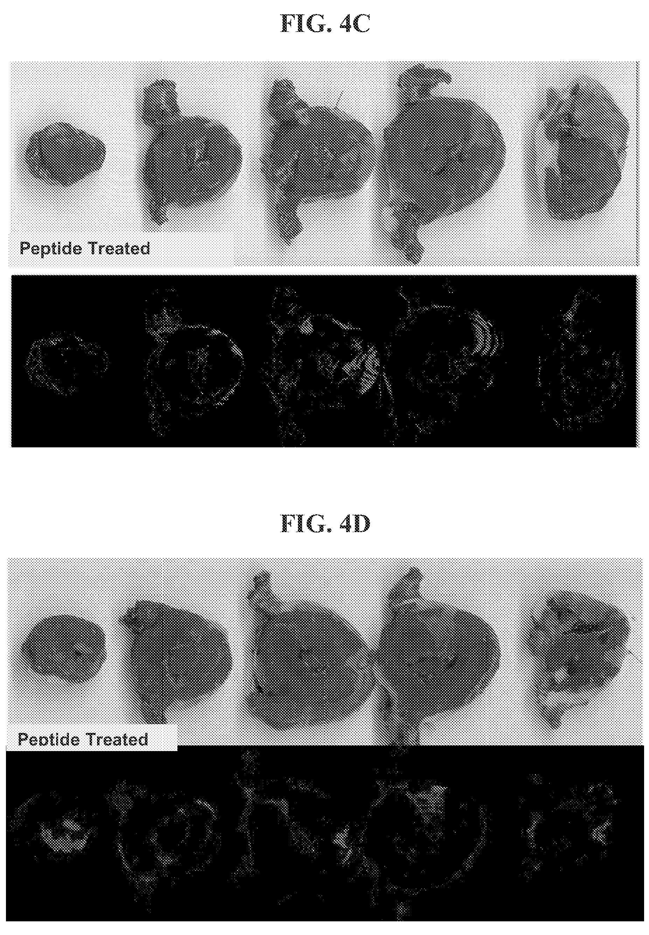

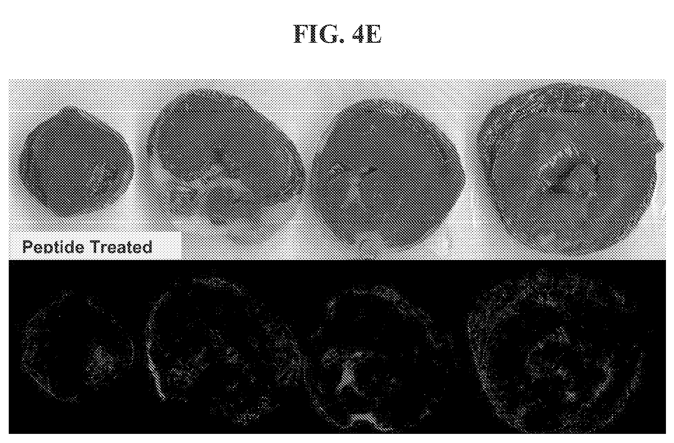

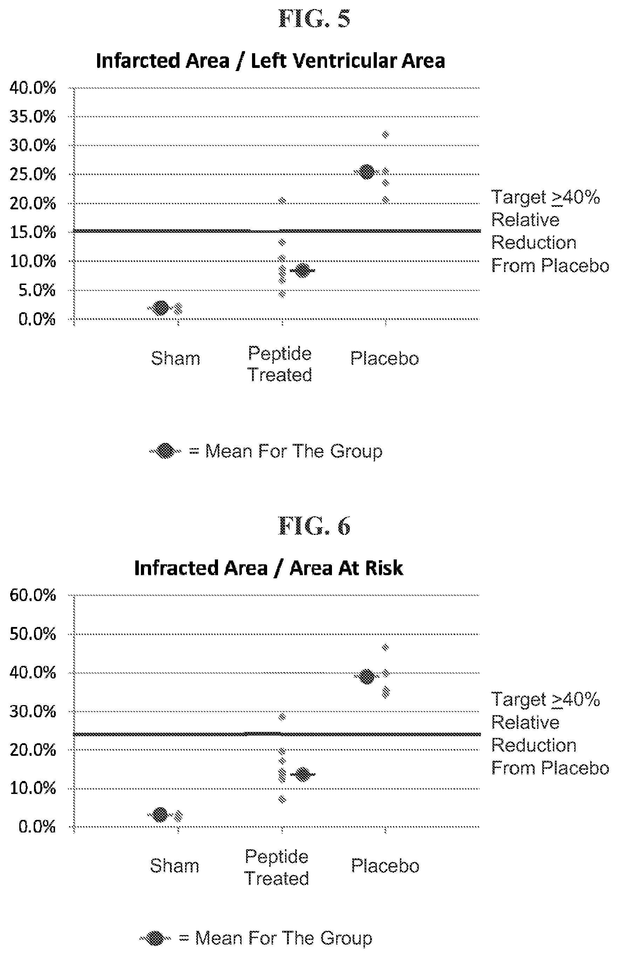

FIGs. 4A, 4B, 4C, 4D, and 4E present data showing infarct size for five different rabbits with induced cardiac ischemia and treated with the peptide. Administration of peptide resulted in decreased infarct size compared to the control. Table 8 presents data showing the ratios of area of risk to left ventricular area infracted area to left ventricular area, and infracted area to area of risk for each of the animals used in this study. FIGs. 5-6 present further data showing the ratios of area of risk to left ventricular area infracted area to left ventricular area, and infracted area to area of risk in peptide-treated and control subjects.

**Table 8. Histopathology Results of Study Animals**

| Group | **Myocardial Area (%)** | | |
|---|---|---|---|
| | **AR/LV** | **IA/LV** | **IA/AR** |
| SHAM (n=2) | 56.0 ± 0.4 | 1.7 ± 0.2 | 2.8 ± 0.3 |
| Peptide (n=10) | 58.2 ± 1.9 | 16.2 ± 3.4 | 24.7 ± 4.7 |
| *% versus placebo* | *6* ± *3* | -*24* ± *16* | *-32* ± *13* |
| Placebo (n=10) | 55.1 ± 2.4 | 21.5 ± 1.7 | 36.1 ± 1.9 |
| ***p value*** (Peptide vs. Placebo) | | p<0.05 | p<0.05 |

These results show that in a standardized rabbit model of acute myocardial ischemia and reperfusion, peptide when administered as an IV continuous infusion beginning at 10 min into a 30 min ischemia period followed by IV continuous infusion for 180 min after reperfusion was able to reduce myocardial infarct size compared to the control group. In the rabbits in which there was a definable response to treatment, the size of the myocardial infarct area was reduced relative to the infarct size noted in control animals. Treatment for less than 3 hours after reperfusion, i.e., 30 min, provided comparable myocardial salvage (data not shown). These results indicate that peptide treatment prevents the occurrence of symptoms of acute cardiac ischemia-reperfusion injury. As such, aromatic-cationic peptides are useful in methods at preventing and treating a vessel occlusion injury in mammalian subjects.

### Example 2. Effects of Peptides in Protecting Against Vessel Occlusion Injury in Humans

This Example will determine whether the administration of D-Arg-2'6'-Dmt-Lys-Phe-NH₂ at the time of revascularization would limit the size of the infarct during acute myocardial infarction.

*Study group*. Men and women, 18 years of age or older, who present after the onset of chest pain, and for whom the clinical decision is made to treat with a revascularization procedure *(e.g.,* PCI or thrombolytics) are eligible for enrollment. The patient may be STEMI or Non-STEMI. A STEMI patient will present with symptoms suggestive of a cutting off of the blood supply to the myocardium and also if the patient's ECG shows the typical heart attack pattern of ST elevation. The diagnosis is made therefore purely on the basis of symptoms, clinical examination and ECG changes. In the case of a Non-ST elevation heart attack, the symptoms of chest pain can be identical to that of a STEMI, but the important difference is that the patient's ECG does not show the typical ST elevation changes traditionally associated with a heart attack. The patient often has a history of having experienced angina, but the ECG at the time of the suspected attack may show no abnormality at all. The diagnosis is suspected on the history and symptoms and is confirmed by a blood test which shows a rise in the concentration of substances called cardiac enzymes in the blood.

*Angiography and Revascularization.* Left ventricular and coronary angiography is performed with the use of standard techniques, just before revascularization. Revascularization is performed by PCI with the use of direct stenting. Alternative revascularization procedures include, but are not limited to, balloon angioplasty; percutaneous transluminal coronary angioplasty; and directional coronary atherectomy

*Experimental Protocol.* After coronary angiography is performed but before the stent is implanted, patients who meet the enrollment criteria are randomly assigned to either the control group or the peptide group. Randomization is performed with the use of a computer-generated randomization sequence. Less than 10 min before direct stenting, the patients in the peptide group receive an intravenous bolus injection of D-Arg-2'6'-Dmt-Lys-Phe-NH₂. The peptide is dissolved in normal saline and is injected through a catheter that is positioned within an antecubital vein. Patients will be equally randomized into any of the following treatment arms (for example, 0, 0.001, 0.005, 0.01, 0.025, 0.05, 0.10, 0.25, 0.5, and 1.0 mg/kg/hour). The peptide will be administered as an IV infusion from about 10 min prior to reperfusion to about 3 hours post-PCI. Following the reperfusion period, the subject may be administered the peptide chronically by any means of administration, *e.g*., subcutaneous or IV injection.

*Infarct Size*. The primary end point is the size of the infarct as assessed by measurements of cardiac biomarkers. Blood samples are obtained at admission and repeatedly over the next 3 days. Coronary biomarkers are measured in each patient. For example, the area under the curve (AUC) (expressed in arbitrary units) for creatine kinase and troponin I release (Beckman kit) may be measured in each patient by computerized planimetry. The principal secondary end point is the size of the infarct as measured by the area of delayed hyperenhancement that is seen on cardiac magnetic resonance imaging (MRI), assessed on day 5 after infarction. For the late-enhancement analysis, 0.2 mmol of gadolinium-tetrazacyclododecanetetraacetic acid (DOTA) per kilogram is injected at a rate of 4 ml per second and was flushed with 15 ml of saline. Delayed hyperenhancement is evaluated 10 min after the injection of gadolinium-DOTA with the use of a three-dimensional inversion-recovery gradient-echo sequence. The images are analyzed in shortaxis slices covering the entire left ventricle.

Myocardial infarction is identified by delayed hyperenhancement within the myocardium, defined quantitatively by an intensity of the myocardial postcontrast signal that is more than 2 SD above that in a reference region of remote, noninfarcted myocardium within the same slice. For all slices, the absolute mass of the infracted area is calculated according to the following formula: infarct mass (in grams of tissue) = ∑ (hyperenhanced area [in square centimeters]) × slice thickness (in centimeters) × myocardial specific density (1.05 g per cubic centimeter).

*Biomarkers to Established Risk Factors.* Levels of N-terminal pro-brain natriuretic peptide (NT-proBNP) and glucose, as well as estimated glomerular filtration rate (eGFR) are measured. These biomarkers all significantly predict all-cause mortality through a median follow-up of about two-and-a-half years. Calculating a risk score based on these three biomarkers can identify patients at high risk of dying during follow-up. It is predicted that the peptide will reduce the risk score of these biomarkers in patients undergoing PCI compared to patients undergoing PCI that do not receive the peptide. Blood samples may be taken for determination of the CK-MB and troponin I. The area under the curve (AUC) (expressed in arbitrary units) for CK-MB and troponin I release can be measured in each patient by computerized planimetry

*Other End Points.* The whole-blood concetration of peptide is immediately prior to PCI as well as at 1, 2, 4, 8 and 12 hours post PCI. Blood pressure and serum concentrations of creatinine and potassium are measured on admission and 24, 48, and 72 hours after PCI. Serum concentrations of bilirubin, γ-glutamyltransferase, and alkaline phosphatase, as well as white-cell counts, are measured on admission and 24 hours after PCI.

The cumulative incidence of major adverse events that occur within the first 48 hours after reperfusion are recorded, including death, heart failure, acute Myocardial infarction, stroke, recurrent ischemia, the need for repeat revascularization, renal or hepatic insufficiency, vascular complications, and bleeding. The infarct-related adverse events are assessed, including heart failure and ventricular fibrillation. In addition, 3 months after acute myocardial infarction, cardiac events are recorded, and global left ventricular function is assessed by echocardiography (Vivid 7 systems; GE Vingmed).

It is predicted that administration of the peptide at the time of reperfusion will be associated with a smaller infarct by some measures than that seen with placebo.

### Example 3. Effects of Peptides in Protecting Against Vessel Occlusion Injury in Humans

This Example will determine whether the administration of D-Arg-2'6'-Dmt-Lys-Phe-NH₂ at the time of revascularization would limit the size of the infarct during acute myocardial infarction.

*Study group*. Men and women, 18 years of age or older, who present with clinical symptoms of AMI, a revascularization procedure are eligible for enrollment. Patients who meet the enrollment criteria are randomly assigned to either the control group or the peptide group. Less than 10 min before direct stenting, the patients in the peptide group receive an intravenous bolus injection of D-Arg-2'6'-Dmt-Lys-Phe-NH₂. The peptide is dissolved in normal saline and is injected through a catheter that is positioned within an antecubital vein. Patients will be equally randomized into any of the following treatment arms (0, 0.001, 0.005, 0.01, 0.025, 0.05, 0.10, 0.25, 0.5, and 1.0 mg/kg/hour). The peptide will be administered as an IV infusion from about 10 min prior to reperfusion to about 3 hours post-revascularization. Following the reperfusion period, the subject may be administered the peptide chronically by any means of administration, *e.g*., subcutaneous or IV injection.

*End Points and Biomarkers*. End points and biomarkers will be measured as described in Example 2. It is predicted that administration of the peptide prior to the time of reperfusion will be associated with a smaller infarct size by some measures than that seen with placebo.

### References

Leshnower BG, Kanemoto S, Matsubara M, Sakamoto H, Hinmon R, Gorman JH 3rd, Gorman RC. Cyclosporine preserves mitochondrial morphology after myocardial ischemia/reperfusion independent of calcineurin inhibition. Ann Thorac Surg., 2008 Oct, 86(4):1286-92.

Zhao L, Roche BM, Wessale JL, Kijtawornrat A, Lolly JL, Shemanski D, Hamlin RL. Chronic xanthine oxidase inhibition following myocardial infarction in rabbits: effects of early versus delayed treatment. Life Sci. 2008 Feb 27;82(9-10):495-502. Epub 2008 Jan 24. PubMed PMID: 18215719.

Hamlin RL, Kijtawornrat A. Use of the rabbit with a failing heart to test for torsadogenicity. Pharmacol Ther. 2008 8 Aug, 119(2):179-85. Epub 2008 Apr 20.

### EQUIVALENTS

The present invention is not to be limited in terms of the particular embodiments described in this application, which are intended as single illustrations of individual aspects of the invention. Many modifications and variations of this invention can be made without departing from its spirit and scope, as will be apparent to those skilled in the art. Functionally equivalent methods and apparatuses within the scope of the invention, in addition to those enumerated herein, will be apparent to those skilled in the art from the foregoing descriptions. Such modifications and variations are intended to fall within the scope of the appended claims. The present invention is to be limited only by the terms of the appended claims, along with the full scope of equivalents to which such claims are entitled. It is to be understood that this invention is not limited to particular methods, reagents, compounds compositions or biological systems, which can, of course, vary. It is also to be understood that the terminology used herein is for the purpose of describing particular embodiments only, and is not intended to be limiting.

In addition, where features or aspects of the disclosure are described in terms of Markush groups, those skilled in the art will recognize that the disclosure is also thereby described in terms of any individual member or subgroup of members of the Markush group.

As will be understood by one skilled in the art, for any and all purposes, particularly in terms of providing a written description, all ranges disclosed herein also encompass any and all possible subranges and combinations of subranges thereof. Any listed range can be easily recognized as sufficiently describing and enabling the same range being broken down into at least equal halves, thirds, quarters, fifths, tenths, *etc.* As a non-limiting example, each range discussed herein can be readily broken down into a lower third, middle third and upper third, *etc*. As will also be understood by one skilled in the art all language such as "up to," "at least," "greater than," "less than," and the like, include the number recited and refer to ranges which can be subsequently broken down into subranges as discussed above. Finally, as will be understood by one skilled in the art, a range includes each individual member. Thus, for example, a group having 1-3 cells refers to groups having 1, 2, or 3 cells. Similarly, a group having 1-5 cells refers to groups having 1, 2, 3, 4, or 5 cells, and so forth.

All patents, patent applications, Provisional applications, and publication referred to or cited herein are incorporated by reference in their entirety, including all figures and tables, to the extent they are not inconsistent with the explicit teachings of this specification.

Other embodiments are set forth within the following claims.

## Claims

1. A composition comprising the peptide D-Arg-2'6'-Dmt-Lys-Phe-NH₂ or a pharmaceutically acceptable salt thereof for use in treating a vessel occlusion injury in a mammalian subject, wherein the subject has undergone, is undergoing or requires a revascularization procedure.

2. The composition for use according to claim 1, for administration prior to the revascularization procedure.

3. The composition for use according to claim 1, for administration after the revascularization procedure.

4. The composition for use according to claim 1, for administration during and after the revascularization procedure.

5. The composition for use according to claim 1, for administration continuously before, during, and after the revascularization procedure.

6. The composition for use according to claim 5, for administration for at least 3 hours after the revascularization procedure; or for administration starting at about 1 hour before the revascularization procedure; or for administration starting at about 30 minutes before the revascularization procedure.

7. The composition for use according to claim 1, wherein the subject is suffering from a myocardial infarction.

8. The composition for use according to claim 1, wherein the subject is suffering from a ST elevation myocardial infarction or a non-ST elevation myocardial infarction.

9. The composition for use according to claim 1, wherein the subject is in need of angioplasty.

10. The composition for use according to claim 1, wherein the revascularization procedure is selected from the group consisting of: balloon angioplasty; insertion of a stent;
percutaneous transluminal coronary angioplasty; or directional coronary atherectomy; or wherein the revascularization procedure comprises-removal of the occlusion.

11. The composition for use according to claim 1, wherein the revascularization procedure comprises administration of one or more thrombolytic agents.

12. The composition for use according to claim 11, wherein the one or more thrombolytic agents are selected from the group consisting of: tissue plasminogen activator; urokinase; prourokinase; streptokinase; acylated form of plasminogen; acylated form of plasmin; and acylated streptokinase-plasminogen complex.

13. The composition for use according to claim 1, wherein the vessel occlusion is a cardiac vessel occlusion; or wherein the vessel occlusion is an intracranial vessel occlusion; or wherein the vessel occlusion is a renal vessel occlusion; or wherein the vessel occlusion is selected from the group consisting of: deep venous thrombosis; peripheral thrombosis; embolic thrombosis; hepatic vein thrombosis; sinus thrombosis; venous thrombosis; an occluded arterio-venal shunt; and an occluded catheter device.

14. The composition for use according to claim 1, wherein the levels of one or more of CK-MB, troponin, N-terminal pro-brain natriuretic peptide (NT-proBNP), glucose, and estimated glomerular filtration rate (eGFR) are reduced in a subject administered the composition relative to a comparable subject undergoing the revascularization procedure, but not administered the composition.

15. The composition for use according to claim 1, wherein the incidence of re-infarction, congestive heart failure, repeat revascularization procedure, renal failure or death in the hospital following the revascularization procedure are reduced in a subject administered the composition relative to a comparable subject undergoing the revascularization procedure, but not administered the composition; or wherein the incidence of Major Adverse Cardiovascular Events, death, cardiac death, or the development of congestive heart failure within 6 months following the revascularization procedure are reduced in a subject administered the composition relative to a comparable subject undergoing the revascularization procedure, but not administered the composition.
